# EUROPEAN PATENT SPECIFICATION

(11) **EP 4 061 961 B1**
(45) Date of publication and mention of the grant of the patent: **08.04.2026**
(21) Application number: 20811708.5
(22) Date of filing: 20.11.2020
(51) Int. Cl.: C12Q 1/6806, C12Q 1/6869

(54) **METHOD FOR DOUBLE STRAND SEQUENCING**
VERFAHREN ZUR DOPPELSTRANGSEQUENZIERUNG
PROCÉDÉ DE SÉQUENÇAGE À DOUBLE BRIN

(30) Priority: 22.11.2019 GB 201917060
(43) Date of publication of application: 28.09.2022
(73) Proprietor: Oxford Nanopore Technologies PLC, Oxford OX4 4DQ (GB)
(72) Inventor: HERON, Andrew John, Oxford Oxfordshire OX4 4DQ (GB); BOWEN, Rebecca Victoria, Oxford Oxfordshire OX4 4DQ (GB); BROWN, Clive Gavin, Oxford Oxfordshire OX4 4DQ (GB)
(74) Representative: J A Kemp LLP
(86) International application number: PCT/GB2020/052972
(87) International publication number: WO 2021/099801

(56) References cited:
- WO-A1-2013/014451
- WO-A1-2016/019360
- US-A1- 2019 271 029

## Description

### Field

The present disclosure relates to methods of sequencing a target double stranded nucleic acid by forming an inter-strand cross-link between the first and second strands of the double stranded nucleic acid. The disclosure also relates to kits, systems and apparatuses for carrying out such methods.

### Background

Nucleic acid sequencing is an increasingly important aspect of medical, biomedical and biotechnological applications. Sequencing of nucleic acids allows the study of genomes and the proteins they encode and, for example, allows correlation between nucleic acid mutations and observable phenomena such as disease indications. Sequencing can be used in evolutionary biology to study the relationship between organisms. Metagenomics involves identifying organisms present in samples, for example microbes in a microbiome, with nucleic acid sequencing allowing the identification of such organisms. In medicine, genetic testing of a subject may highlight the risk of genetic disease or allow the selection of optimum therapies to treat medical conditions. DNA sequencing is also a key technology in forensic science. In view of the many and varied applications of DNA sequencing, there is a need for improved methods for sequencing nucleic acids, particularly double stranded nucleic acids.

Nucleic acids can be sequenced using many known technologies. Single molecule techniques have proven to be particularly attractive due to their high fidelity, avoidance of amplification bias, and the possibility of achieving extremely long read lengths. Single molecule sequencing can also provide information on the presence of characteristics such as base modifications, oxidation, reduction, decarboxylation, deamination and more.

One attractive method of single molecule sequencing of nucleic acids is nanopore sensing. Nanopore sensing is an approach to analyte detection and characterization that relies on the observation of individual binding or interaction events between the analyte molecules and an ion conducting channel. Nanopore sensors can be created by placing a single pore of nanometre dimensions in an electrically insulating membrane and measuring voltage-driven ion currents through the pore in the presence of analyte molecules. The presence of an analyte inside or near the nanopore will alter the ionic flow through the pore, resulting in altered ionic or electric currents being measured over the channel. The identity of an analyte is revealed through its distinctive current signature, notably the duration and extent of current blocks and the variance of current levels during its interaction time with the pore. Nanopore sensing has the potential to allow rapid and cheap polynucleotide sequencing, providing single molecule sequence reads of polynucleotides of tens to tens of thousands bases length.

Other known methods of single molecule sequencing of nucleic acids include single-molecule real-time sequencing based on the detection of base incorporation catalysed by enzymes such as DNA polymerases. In one known method, a polymerase catalyses the formation of a complementary strand to a target nucleic acid strand to be sequenced using labelled nucleotides. The sequential incorporation of the labelled nucleotides into the strand being synthesized releases the labels (e.g. fluorescent labels) attached to each nucleotide and detection of the label allows the identification of the nucleotide, and thus the sequence of the template strand, to be determined.

Single molecule sequencing of nucleic acids typically relies on the controlled processing of the polynucleotide. For example, in nanopore sensing of polynucleotides, it is important to control the movement of the polynucleotide with respect to the pore. Uncontrolled movement can prevent or impede accurate characterisation of the polynucleotides. For example, accurately distinguishing each nucleotide in a homopolymeric polynucleotide is problematic when the movement of the polynucleotide with respect to the pore is not controlled. Similarly, in sequencing-by-synthesis techniques, it is important to control the synthesis reaction with respect to the sensor used. Uncontrolled movement can prevent or impede accurate characterisation of the polynucleotide as bases may be skipped or processed too quickly for the signal to be readily deconvoluted.

To address this problem, it is known to use a polynucleotide-processing enzyme, also in some embodiments known as a motor protein, to control the movement of a polymer such as a polynucleotide whilst it is characterised. Suitable enzymes include polynucleotide-handling enzymes such as helicases, exonucleases, topoisomerases and the like. The protein processes the polynucleotide in a controlled manner. For example, in nanopore sequencing, a motor protein can be used to control the movement of a polymer such as a polynucleotide with respect to the pore. In single-molecule real-time sequencing the polymerase controls the movement of the target polynucleotide as it moves with respect to the polymerase as it is processed.

One challenge in single molecule sequencing of nucleic acids is that of improving base calling accuracy. In conventional techniques, only one strand of a double stranded nucleic acid (e.g. the template) is sequenced, with the other strand (e.g. the complement) being either omitted from the sequencing reaction, or with the information from that strand being discarded. However, the present inventors have recognised that sequencing both strands of a double stranded polynucleotide can provide valuable orthogonal proof-reading information. Thus, the sequencing information obtained for the first strand of a double stranded nucleic acid can be supplemented by the sequencing information obtained for the second strand of the double stranded nucleic acid.

There are various methods known in the art to allow both strands of a double stranded nucleic acid to be sequentially sequenced. One known method comprises attaching to the double stranded nucleic acid a so-called hairpin adapter. The hairpin adapter links the two strands of the nucleic acid together by their 5' and 3' ends so that they form a single contiguous strand which can be sequenced using single molecule techniques.

Whilst the use of hairpin adapters has led to some advantages, there remains a need for new methods of sequencing both strands of a double stranded nucleic acid. For example, modification of the 3' or 5' ends of the double stranded nucleic acid to be sequenced can impede attachment of a hairpin adapter. The requirement for the hairpin adapter itself can lead to increased complexity in the experimental design and may require dedicated ligation reagents. The requirement to attach the adapter may increase sample preparation time or complexity, risking sample contamination or degradation. Accordingly, methods of sequencing both strands of a double stranded nucleic acid which do not require a hairpin adapter would be valuable.

### Summary

The disclosure relates to methods of sequencing a double stranded nucleic acid. The methods comprise forming an inter-strand cross-link between the first and second strands of the double stranded nucleic acid. The first and second strands of the double stranded nucleic acid are often referred to as the template and complement, respectively.

The methods disclosed herein comprise forming an inter-strand cross-link between the first and second strands of the double stranded nucleic acid by exposing the double stranded nucleic acid to a cross-linking agent. In some embodiments, the methods further comprise allowing the target double stranded nucleic acid to break at or in the vicinity of the inter-strand cross-link which is formed by the cross-linking agent. The break leads to the formation of at least one double stranded nucleic acid construct which is cross-linked at or in the vicinity of the terminus of the construct. The methods comprise sequencing the double stranded nucleic acid construct thus formed using a single molecule sequencing technique.

Accordingly, provided herein is a method of sequencing a target double stranded nucleic acid comprising:
(a) forming an inter-strand cross-link between the first and second strands of the target double stranded nucleic acid by exposing the target double stranded nucleic acid to a cross-linking agent; thereby forming at least one cross-linked double stranded nucleic acid construct; and
(b) sequencing the cross-linked construct formed in (a) using a single molecule sequencing technique.

In some embodiments, the method further comprises allowing the double stranded nucleic acid to break at or in the vicinity of the inter-strand cross-link formed in step (a) so that the inter-strand cross-link is positioned at or in the vicinity of the terminus of the construct

In some embodiments, the break at or in the vicinity of the inter-strand cross-link occurs naturally due to distortion. In some embodiments, the break at or in the vicinity of the inter-strand cross-link is induced by physical agitation.

In some embodiments, the single molecule sequencing technique comprises contacting the cross-linked construct with an enzyme which sequentially processes the first strand and the second strand of the cross-linked construct formed. In some embodiments, the sequencing technique provides orthogonal proof-reading sequence information.

In some embodiments, the cross-linking agent is or comprises electromagnetic radiation. In some embodiments, the cross-linking agent is or comprises a chemical reagent. In some embodiments, the cross-linking agent is or comprises a nucleic acid cross-linking enzyme.

In some embodiments, the inter-strand cross-link is formed at a random position on the target double stranded nucleic acid. In some embodiments, the inter-strand cross-link is targeted to a particular base composition on the target double stranded nucleic acid.

In some embodiments, the inter-strand cross-link is formed between nucleobases in the first and second strands of the target double stranded nucleic acid. In some embodiments, the inter-strand cross-link is formed between sugar groups in the first and second strands of the target double stranded nucleic acid. In some embodiments, the interstrand cross-link is formed between nucleobases in the first and second strands of the target double stranded nucleic acid and between sugar groups in the first and second strands of the target double stranded nucleic acid. In some embodiments, the inter-strand cross-link is formed between nucleobase adducts in the first and second strands of the target double stranded nucleic acid.

In some embodiments, the cross-link is positioned within 100 bases of the terminus of the cross-linked construct formed in part (b) of claim 1.

In some embodiments, the single molecule sequencing technique comprises sequencing using a nanopore sensor.

Also provided herein are cross-linked nucleic acid constructs formed by exposing a double stranded nucleic acid to a cross-linking agent as described herein.

Also provided are systems comprising a cross-linked nucleic acid constructs formed by exposing a double stranded nucleic acid to a cross-linking agent as described herein; one or more polynucleotide-processing enzymes as described herein and one or more nanopores.

Also provided are kits comprising one or more cross-linking reagents and one or more polynucleotide-processing enzymes.

### Brief Description of the Figures

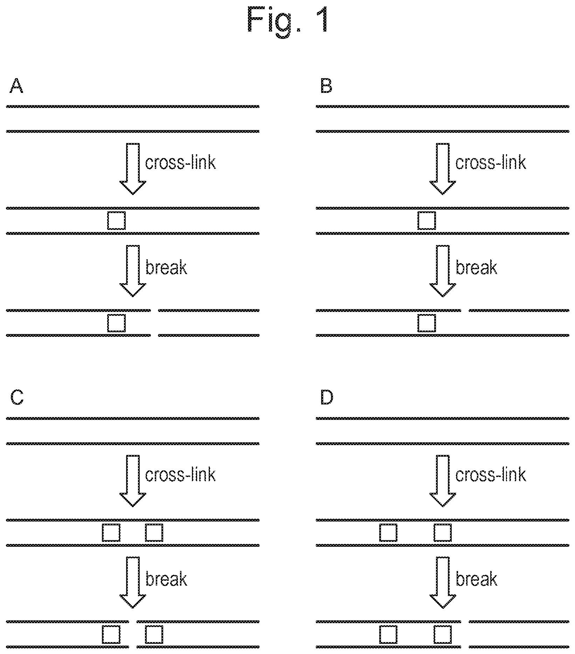

**Figure** 1. Schematic of the formation of inter-strand cross-links in a double stranded nucleic acid, followed by optional breaking of the inter-strand cross-linked double stranded nucleic acid to form at least one inter-strand cross-linked double stranded construct. **A:** One inter-strand cross-link can be formed in the double stranded nucleic acid and both strands of the double stranded nucleic acid allowed to break at or in the vicinity of the inter-strand cross-link to form an inter-strand cross-linked construct and a non-crosslinked double stranded polynucleotide. **B:** One inter-strand cross-link can be formed in the double stranded nucleic acid and one strand of the double stranded nucleic acid allowed to break at or in the vicinity of the inter-strand cross-link to form a inter-strand cross-linked construct and a single stranded polynucleotide. **C:** A plurality of inter-strand cross-links (of which two inter-strand cross-links are shown) can be formed in the double stranded nucleic acid and both strands of the double stranded nucleic acid allowed to break between two of the plurality of inter-strand cross-links to form two inter-strand cross-linked constructs. **D:** A plurality of inter-strand cross-links (of which two inter-strand cross-links are shown) can be formed in the double stranded nucleic acid and both strands of the double stranded nucleic acid allowed to break at or in the vicinity of one of the plurality of inter-strand cross-links to form an inter-strand cross-linked construct and a non-crosslinked double stranded polynucleotide.

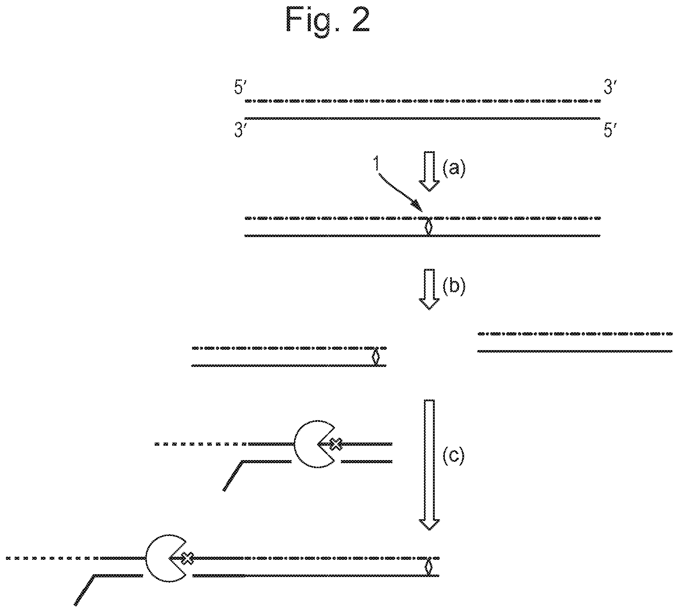

**Figure 2****.** Schematic illustrating cross-linking and preparing double stranded nucleic acids for single-molecule sequencing. **Step (a):** a double-stranded polynucleotide (e.g. dsDNA) is exposed to conditions that create an inter-strand crosslink between the two strands of the double-strand substrate (e.g. by covalently cross-linking adjacent bases of the strands). **Step (b):** The cross-linked substrates may be prepared for sequencing, e.g. nanopore sequencing, by allowing the strand to break at or near the crosslink site. **Step (c):** Sequencing adapters may be attached to facilitate sequencing of the inter-strand crosslinked construct (eg. by ligation or transposition). By way of non-limiting example, the figure illustrates preparing the cross-linked substrate with an dA-tailing method, which leaves a 3' dA-tail, to which a complementary sequencing adapter can be attached by ligation as shown.

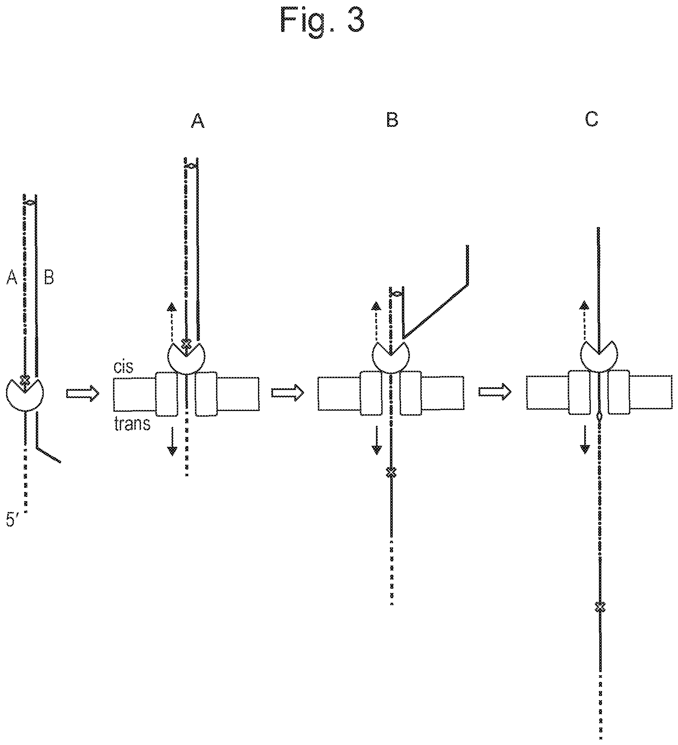

**Figure 3****.** Schematic illustrating a method of sequencing the inter-strand cross-linked construct obtained as described in Figure 2 using a nanopore system. By way of non-limiting example, capture and translocation of a cross-linked double-stranded polynucleotide through a nanopore in a membrane, with movement controlled by a motor protein, is illustrated. **Step (A):** The substrate is captured in the nanopore from the cis side (e.g. under the force of an applied voltage across the membrane) until the motor enzyme is brought into contact with the top of the nanopore. **Step (B):** The motor protein proceeds through a stall region of the sequencing adapter and begins to control the movement of the polynucleotide through the nanopore from the cis side to the trans side. The motor protein first proceeds through the template part of the double-strand molecule ("section A"), then **[Step (C)]** proceeds past the cross-link connecting the two strands, then controls the movement of the second reverse-complement section ("section B") through the nanopore.

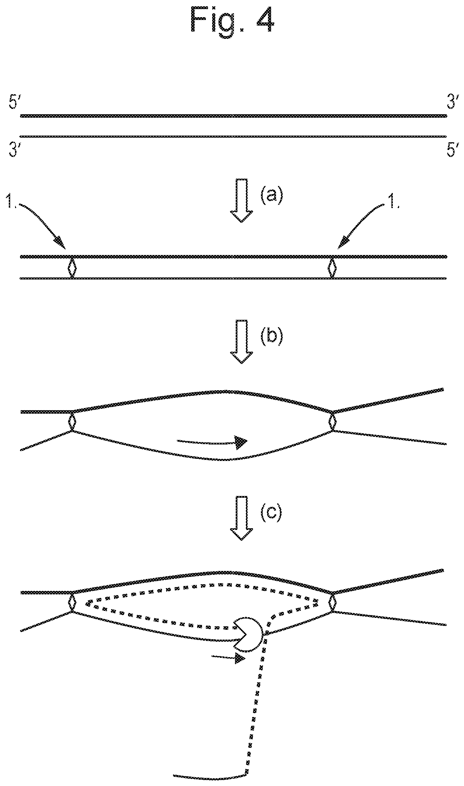

**Figure 4****.** Schematic illustrating how a double stranded nucleic acid (e.g. double-stranded DNA) can be cross-linked in multiple locations and then used to prepare a repeating circular synthesis product. **Step (a):** A double stranded nucleic acid (e.g. double-strand DNA strand) is cross-linked in two sites, creating a "circular" region bounded by the two inter-strand cross-links. **Step (b):** The substrate can be processed in a number of ways, for example melting and invading primers (strand with arrow head). **Step (c):** polymerase mediated strand copying can be initiated (dotted strand). The polymerase cannot proceed through the inter-strand cross-link sites whilst remaining on the same strand, so when encountering the inter-strand cross-links the polymerase switches to the adjacent strand (the synthesized strand thus becomes complementary to the adjacent strand), and proceeds to replicate around the "circular" region as shown. The strand product of synthesis can be then be processed and sequenced, for example by attaching sequencing adapters and sequencing on a nanopore system.

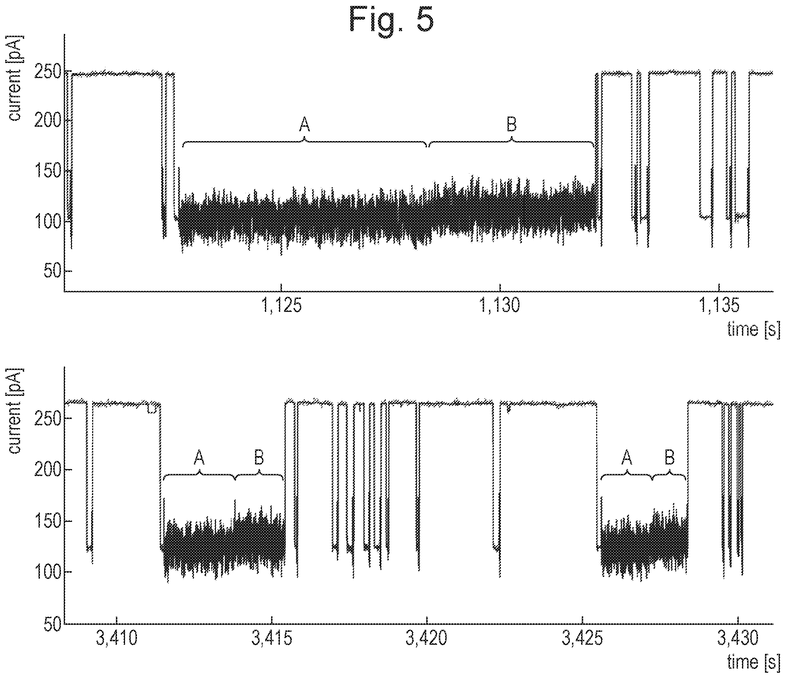

**Figure 5****.** Exemplary electrical current (*y* axis) vs. time (*x* axis) data from single nanopore sequencing of a lambda DNA sample using baseline sequencing parameters on an Oxford Nanopore MinION nanopore sequencer. Two typical sections of data from a single experiment are shown. Prior to sequencing the sample was cross-linked as described in the Example. The nanopores had open-pore currents of approximately 250 pA that block down to a lower current upon capture of DNA analytes. Short-lived events are from free (unbound) sequencing adapter in the sample, long-lived events characteristic of DNA sequencing are marked with sections A and B, which correspond to the template (A) and linked reverse-complement (B) sections of cross-linked dsDNA substrates. The data is discussed in the example.

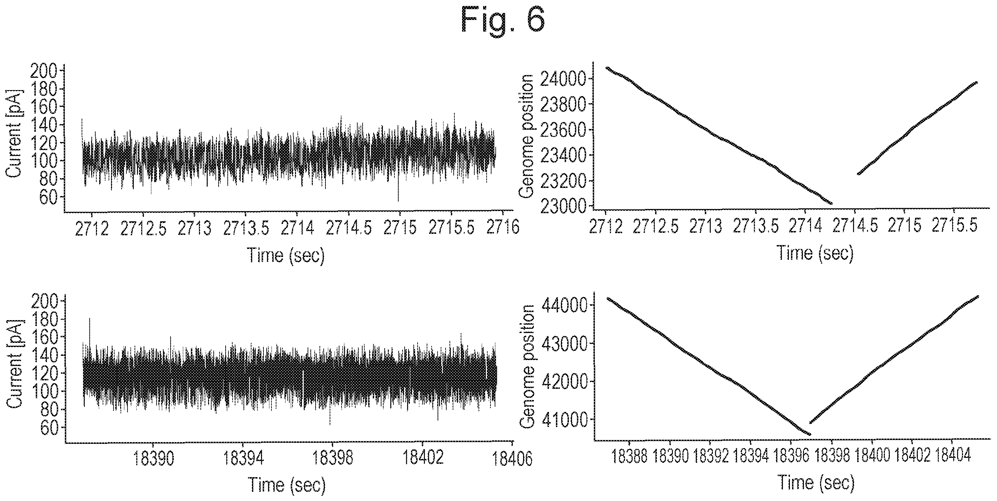

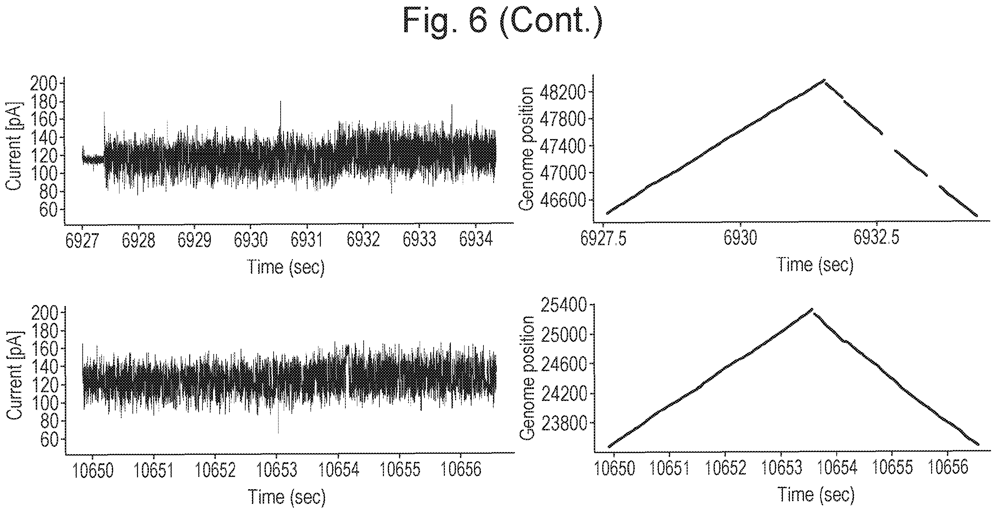

**Figure 6****.** Exemplary electrical current (y axis) vs. time (x axis) data (left column) obtained from single nanopore sequencing of cross-linked lambda DNA using baseline sequencing parameters on an Oxford Nanopore MinION nanopore sequencer. The left panel shows the current vs. time signal, and the right panel shows the alignment of the basecalls for these strands aligned to the Lambda Phage genome reference. The data is discussed in the example.

### Detailed Description

The present invention will be described with respect to particular embodiments and with reference to certain drawings but the invention is not limited thereto but only by the claims. Any reference signs in the claims shall not be construed as limiting the scope. Of course, it is to be understood that not necessarily all aspects or advantages may be achieved in accordance with any particular embodiment of the invention. Thus, for example those skilled in the art will recognize that the invention may be embodied or carried out in a manner that achieves or optimizes one advantage or group of advantages as taught herein without necessarily achieving other aspects or advantages as may be taught or suggested herein.

The invention, both as to organization and method of operation, together with features and advantages thereof, may best be understood by reference to the following detailed description when read in conjunction with the accompanying drawings. The aspects and advantages of the invention will be apparent from and elucidated with reference to the embodiment(s) described hereinafter. Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment.

It should be appreciated that "embodiments" of the disclosure can be specifically combined together unless the context indicates otherwise. The specific combinations of all disclosed embodiments (unless implied otherwise by the context) are further disclosed embodiments of the claimed invention.

In addition as used in this specification and the appended claims, the singular forms "a", "an", and "the" include plural referents unless the content clearly dictates otherwise. Thus, for example, reference to "a polynucleotide" includes two or more polynucleotides, reference to "a motor protein" includes two or more such proteins, reference to "a helicase" includes two or more helicases, reference to "a monomer" refers to two or more monomers, reference to "a pore" includes two or more pores and the like.

### Definitions

Where an indefinite or definite article is used when referring to a singular noun e.g. "a" or "an", "the", this includes a plural of that noun unless something else is specifically stated. Where the term "comprising" is used in the present description and claims, it does not exclude other elements or steps. Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. The following terms or definitions are provided solely to aid in the understanding of the invention. Unless specifically defined herein, all terms used herein have the same meaning as they would to one skilled in the art of the present invention. Practitioners are particularly directed to Sambrook et al., Molecular Cloning: A Laboratory Manual, 4th ed., Cold Spring Harbor Press, Plainsview, New York (2012); and Ausubel et al., Current Protocols in Molecular Biology (Supplement 114), John Wiley & Sons, New York (2016), for definitions and terms of the art. The definitions provided herein should not be construed to have a scope less than understood by a person of ordinary skill in the art.

"About" as used herein when referring to a measurable value such as an amount, a temporal duration, and the like, is meant to encompass variations of ± 20 % or ± 10 %, more preferably ± 5 %, even more preferably ± 1 %, and still more preferably ± 0.1 % from the specified value, as such variations are appropriate to perform the disclosed methods.

"Nucleotide sequence", "DNA sequence" or "nucleic acid molecule(s)" as used herein refers to a polymeric form of nucleotides of any length, either ribonucleotides or deoxyribonucleotides. This term refers only to the primary structure of the molecule. Thus, this term includes double- and single-stranded DNA, and RNA. The term "nucleic acid" as used herein, is a single or double stranded covalently-linked sequence of nucleotides in which the 3' and 5' ends on each nucleotide are joined by phosphodiester bonds. The polynucleotide may be made up of deoxyribonucleotide bases or ribonucleotide bases. Nucleic acids may be manufactured synthetically in vitro or isolated from natural sources. Nucleic acids may further include modified DNA or RNA, for example DNA or RNA that has been methylated, or RNA that has been subject to post-translational modification, for example 5'-capping with 7-methylguanosine, 3'-processing such as cleavage and polyadenylation, and splicing. Nucleic acids may also include synthetic nucleic acids (XNA), such as hexitol nucleic acid (HNA), cyclohexene nucleic acid (CeNA), threose nucleic acid (TNA), glycerol nucleic acid (GNA), locked nucleic acid (LNA) and peptide nucleic acid (PNA). Sizes of nucleic acids, also referred to herein as "polynucleotides" are typically expressed as the number of base pairs (bp) for double stranded polynucleotides, or in the case of single stranded polynucleotides as the number of nucleotides (nt). One thousand bp or nt equal a kilobase (kb). Polynucleotides of less than around 40 nucleotides in length are typically called "oligonucleotides" and may comprise primers for use in manipulation of DNA such as via polymerase chain reaction (PCR).

The term "amino acid" in the context of the present disclosure is used in its broadest sense and is meant to include organic compounds containing amine (NH₂) and carboxyl (COOH) functional groups, along with a side chain (e.g., a R group) specific to each amino acid. In some embodiments, the amino acids refer to naturally occurring L α-amino acids or residues. The commonly used one and three letter abbreviations for naturally occurring amino acids are used herein: A=Ala; C=Cys; D=Asp; E=Glu; F=Phe; G=Gly; H=His; I=Ile; K=Lys; L=Leu; M=Met; N=Asn; P=Pro; Q=Gln; R=Arg; S=Ser; T=Thr; V=Val; W=Trp; and Y=Tyr (Lehninger, A. L., (1975) Biochemistry, 2d ed., pp. 71-92, Worth Publishers, New York). The general term "amino acid" further includes D-amino acids, retro-inverso amino acids as well as chemically modified amino acids such as amino acid analogues, naturally occurring amino acids that are not usually incorporated into proteins such as norleucine, and chemically synthesised compounds having properties known in the art to be characteristic of an amino acid, such as β-amino acids. For example, analogues or mimetics of phenylalanine or proline, which allow the same conformational restriction of the peptide compounds as do natural Phe or Pro, are included within the definition of amino acid. Such analogues and mimetics are referred to herein as "functional equivalents" of the respective amino acid. Other examples of amino acids are listed by Roberts and Vellaccio, The Peptides: Analysis, Synthesis, Biology, Gross and Meiehofer, eds., Vol. 5 p. 341, Academic Press, Inc., N.Y. 1983,

The terms "polypeptide", and "peptide" are interchangeably used herein to refer to a polymer of amino acid residues and to variants and synthetic analogues of the same. Thus, these terms apply to amino acid polymers in which one or more amino acid residues is a synthetic non-naturally occurring amino acid, such as a chemical analogue of a corresponding naturally occurring amino acid, as well as to naturally-occurring amino acid polymers. Polypeptides can also undergo maturation or post-translational modification processes that may include, but are not limited to: glycosylation, proteolytic cleavage, lipidization, signal peptide cleavage, propeptide cleavage, phosphorylation, and such like. A peptide can be made using recombinant techniques, e.g., through the expression of a recombinant or synthetic polynucleotide. A recombinantly produced peptide it typically substantially free of culture medium, e.g., culture medium represents less than about 20 %, more preferably less than about 10 %, and most preferably less than about 5 % of the volume of the protein preparation.

The term "protein" is used to describe a folded polypeptide having a secondary or tertiary structure. The protein may be composed of a single polypeptide, or may comprise multiple polypepties that are assembled to form a multimer. The multimer may be a homooligomer, or a heterooligmer. The protein may be a naturally occurring, or wild type protein, or a modified, or non-naturally, occurring protein. The protein may, for example, differ from a wild type protein by the addition, substitution or deletion of one or more amino acids.

A "variant" of a protein encompass peptides, oligopeptides, polypeptides, proteins and enzymes having amino acid substitutions, deletions and/or insertions relative to the unmodified or wild-type protein in question and having similar biological and functional activity as the unmodified protein from which they are derived. The term "amino acid identity" as used herein refers to the extent that sequences are identical on an amino acidby-amino acid basis over a window of comparison. Thus, a "percentage of sequence identity" is calculated by comparing two optimally aligned sequences over the window of comparison, determining the number of positions at which the identical amino acid residue (e.g., Ala, Pro, Ser, Thr, Gly, Val, Leu, Ile, Phe, Tyr, Trp, Lys, Arg, His, Asp, Glu, Asn, Gln, Cys and Met) occurs in both sequences to yield the number of matched positions, dividing the number of matched positions by the total number of positions in the window of comparison (i.e., the window size), and multiplying the result by 100 to yield the percentage of sequence identity.

For all aspects and embodiments of the present invention, a "variant" has at least 50%, 60%, 70%, 80%, 90%, 95% or 99% complete sequence identity to the amino acid sequence of the corresponding wild-type protein. Sequence identity can also be to a fragment or portion of the full length polynucleotide or polypeptide. Hence, a sequence may have only 50 % overall sequence identity with a full length reference sequence, but a sequence of a particular region, domain or subunit could share 80 %, 90 %, or as much as 99 % sequence identity with the reference sequence.

The term "wild-type" refers to a gene or gene product isolated from a naturally occurring source. A wild-type gene is that which is most frequently observed in a population and is thus arbitrarily designed the "normal" or "wild-type" form of the gene. In contrast, the term "modified", "mutant" or "variant" refers to a gene or gene product that displays modifications in sequence (e.g., substitutions, truncations, or insertions), post-translational modifications and/or functional properties (e.g., altered characteristics) when compared to the wild-type gene or gene product. It is noted that naturally occurring mutants can be isolated; these are identified by the fact that they have altered characteristics when compared to the wild-type gene or gene product. Methods for introducing or substituting naturally-occurring amino acids are well known in the art. For instance, methionine (M) may be substituted with arginine (R) by replacing the codon for methionine (ATG) with a codon for arginine (CGT) at the relevant position in a polynucleotide encoding the mutant monomer. Methods for introducing or substituting non-naturally-occurring amino acids are also well known in the art. For instance, non-naturally-occurring amino acids may be introduced by including synthetic aminoacyltRNAs in the IVTT system used to express the mutant monomer. Alternatively, they may be introduced by expressing the mutant monomer in *E. coli* that are auxotrophic for specific amino acids in the presence of synthetic (i.e. non-naturally-occurring) analogues of those specific amino acids. They may also be produced by naked ligation if the mutant monomer is produced using partial peptide synthesis. Conservative substitutions replace amino acids with other amino acids of similar chemical structure, similar chemical properties or similar side-chain volume. The amino acids introduced may have similar polarity, hydrophilicity, hydrophobicity, basicity, acidity, neutrality or charge to the amino acids they replace. Alternatively, the conservative substitution may introduce another amino acid that is aromatic or aliphatic in the place of a pre-existing aromatic or aliphatic amino acid. Conservative amino acid changes are well-known in the art and may be selected in accordance with the properties of the 20 main amino acids as defined in Table 1 below. Where amino acids have similar polarity, this can also be determined by reference to the hydropathy scale for amino acid side chains in Table 2.

**Table 1 - Chemical properties of amino acids**

| | | | |
|---|---|---|---|
| Ala | aliphatic, hydrophobic, neutral | Met | hydrophobic, neutral |
| Cys | polar, hydrophobic, neutral | Asn | polar, hydrophilic, neutral |
| Asp | polar, hydrophilic, charged (-) | Pro | hydrophobic, neutral |
| Glu | polar, hydrophilic, charged (-) | Gln | polar, hydrophilic, neutral |
| Phe | aromatic, hydrophobic, neutral | Arg | polar, hydrophilic, charged (+) |
| Gly | aliphatic, neutral | Ser | polar, hydrophilic, neutral |
| His | aromatic, polar, hydrophilic, charged (+) | Thr | polar, hydrophilic, neutral |
| Ile | aliphatic, hydrophobic, neutral | Val | aliphatic, hydrophobic, neutral |
| Lys | polar, hydrophilic, charged(+) | Trp | aromatic, hydrophobic, neutral |
| Leu | aliphatic, hydrophobic, neutral | Tyr | aromatic, polar, hydrophobic |

**Table 2 - Hydropathy scale**

| Side Chain | Hydropathy |
|---|---|
| Ile | 4.5 |
| Val | 4.2 |
| Leu | 3.8 |
| Phe | 2.8 |
| Cys | 2.5 |
| Met | 1.9 |
| Ala | 1.8 |
| Gly | -0.4 |
| Thr | -0.7 |
| Ser | -0.8 |
| Trp | -0.9 |
| Tyr | -1.3 |
| Pro | -1.6 |
| His | -3.2 |
| Glu | -3.5 |
| Gln | -3.5 |
| Asp | -3.5 |
| Asn | -3.5 |
| Lys | -3.9 |
| Arg | -4.5 |

A mutant or modified protein, monomer or peptide can also be chemically modified in any way and at any site. A mutant or modified monomer or peptide is preferably chemically modified by attachment of a molecule to one or more cysteines (cysteine linkage), attachment of a molecule to one or more lysines, attachment of a molecule to one or more non-natural amino acids, enzyme modification of an epitope or modification of a terminus. Suitable methods for carrying out such modifications are well-known in the art. The mutant of modified protein, monomer or peptide may be chemically modified by the attachment of any molecule. For instance, the mutant of modified protein, monomer or peptide may be chemically modified by attachment of a dye or a fluorophore.

### Disclosed Methods

The disclosure relates to methods of sequencing target double stranded nucleic acids by forming an inter-strand cross-link between the first and second strands of the target double stranded nucleic acid, and sequencing the cross-linked construct formed.

The inventors have surprisingly found that polynucleotide processing enzymes, also in some embodiments known as motor proteins, can be capable of sequentially processing the first and second strands of double stranded nucleic acids when the first and second strands are linked by a inter-strand cross-link. The inventors surprisingly found that it is not required that the 5' and 3' ends of the double stranded nucleic acid are joined together. Rather than simply proceeding from the first strand of a double stranded nucleic acid to the second strand via a polynucleotide bridge such as provided by a hairpin adaptor, the inventors have surprisingly found that polynucleotide processing enzymes are capable of processing from the first to the second strand of a double stranded polynucleotide at an inter-strand cross-link. The methods provided herein exploit this ability. For example, by sequencing a cross-linked construct obtained by forming an inter-strand cross-link between the first and second strands of a target double stranded nucleic acid, the sequential processing of the first and second strand can provide orthogonal proof-reading sequence information. Polynucleotide-processing enzymes are described in more detail herein.

Accordingly, provided herein is a method of sequencing a target double stranded nucleic acid comprising:
(a) forming an inter-strand cross-link between the first and second strands of the target double stranded nucleic acid by exposing the target double stranded nucleic acid to a crosslinking agent; thereby forming at least one cross-linked double stranded nucleic acid construct; and
(b) sequencing the cross-linked construct formed in (a) using a single molecule sequencing technique.

Inter-strand cross-links between the first and second strand can be formed by any suitable method. Some exemplary means are described in more detail herein.

Typically, in the disclosed methods, the inter-strand cross-link is not a link between the 5' and 3' ends of a double stranded nucleic acid. For example, as used herein, an interstrand cross-link is not the result of using a hairpin adaptor. While a hairpin adaptor does connect the first and second strands of a double stranded nucleic acid, it does not form an inter-strand cross-link as used herein.

An inter-strand cross-link may be formed at any suitable position on the double stranded nucleic acid strand. For example, the inter-strand cross-link may be formed at a random position on the double stranded nucleic acid. Alternatively, the inter-strand crosslink may be targeted to a particular base composition on the double stranded nucleic acid. In other words, in some embodiments the disclosed methods may comprise forming the inter-strand cross-link at a random position. In other embodiments the disclosed methods comprise determining the location of the inter-strand cross-link using the base composition of the double stranded nucleic acid. The base composition can be selected, determined or controlled to be a particular composition for targeting the inter-strand cross-link.

In some embodiments, the base composition can be determined in the double stranded nucleic acid by introducing a target sequence into the double stranded nucleic acid. The target sequence can be pre-determined to be susceptible to formation of an interstrand cross-link when contacted with a cross-linking agent. A target sequence can be introduced into a double stranded nucleic acid using any suitable technique. For example, a target sequence can be introduced into a genome using CRISPR, e.g. by using CRISPR-Cas9 genome editing. A target sequence can be introduced into a double stranded nucleic acid using conventional cloning techniques such as those described in Sambrook et al., Molecular Cloning: A Laboratory Manual, 4th ed., Cold Spring Harbor Press, Plainsview, New York (2012); and Ausubel et al., Current Protocols in Molecular Biology (Supplement 114), John Wiley & Sons, New York (2016). Other suitable techniques will be apparent to those skilled in the art.

In some embodiments the methods comprise attaching one or more adaptors to the target double stranded nucleic acid (e.g. by ligation). In such embodiments the disclosed methods may comprise forming the inter-strand cross-link in the adaptor.

Those skilled in the art will appreciate that the location of the inter-strand cross-link will determine the sequencing results that are obtained. Thus, the location of the interstrand cross-link can be controlled to provide the desired information. For example, if it is desired to sequence only the initial portion of a long double stranded nucleic acid, then an inter-strand cross-link can be introduced downstream of that initial portion. The disclosed methods will allow the selective sequencing of that portion of the double stranded nucleic acid. On the other hand, if it is desired to sequence the entirety of a double stranded nucleic acid, then the inter-strand cross-link can be introduced at the end of the double stranded nucleic acid. The disclosed methods will then allow the selective sequencing of the entire double stranded nucleic acid.

The random positioning of inter-strand cross-links can also be used to provide useful information. For example, a population of double stranded nucleic acids can be subjected to the inter-strand cross-linking reaction conditions. The characteristic distribution of inter-strand cross-links will give rise to a population of polynucleotide sequences of specific lengths. The distribution of such lengths may thus be characteristic for the population of double stranded nucleic acids. In this way, different populations can be compared. Alternatively, the prevalence of inter-strand cross-links can provide information regarding the sequence of the double stranded nucleic acid prior to the formation of the inter-strand cross-links.

In another embodiment, the random positioning of inter-strand cross-links can be useful in genomic sequencing. Incorporation of random inter-strand cross-links (e.g. at a low level across the double stranded nucleic acid) can allow an equal balance of reads (i.e. sequencing information) to be obtained from across the genome.

These and other advantages will be apparent to those skilled in the art.

As discussed in more detail below, some embodiments of the disclosed methods comprise allowing the target double stranded nucleic acid to break at or in the vicinity of the inter-strand cross-link. As described in more detail herein, the break may naturally occur as a result of the inter-strand cross-link. For example, in some embodiments the inter-strand cross-link causes distortion in the structure of the target double stranded nucleic acid and the distortion causes the target double stranded nucleic acid to break at or in the vicinity of the inter-strand cross-link. In other embodiments the methods disclosed herein may comprise causing the target double stranded nucleic acid to break at or in the vicinity of the inter-strand cross-link. For example, in some embodiments the methods comprise physically agitating the cross-linked double stranded nucleic acid to cause the double stranded nucleic acid to break at or in the vicinity of the inter-strand cross-link. Breaking of the target double stranded nucleic acid at or in the vicinity of the inter-strand cross-link, as described in more detail herein, forms a double stranded nucleic acid construct cross-linked at or in the vicinity of the terminus of the construct.

As described in more detail herein, the disclosed methods comprise sequencing the cross-linked double stranded nucleic acid construct using a single molecule sequencing technique. Any suitable sequencing technique can be used. Exemplary suitable sequencing techniques are discussed in more detail herein. For example, in some preferred embodiments, the sequencing technique is a nanopore sensing method. Nanopore sensing methods are described in detail here. However, the methods disclosed herein are not limited to nanopore sensing. Other single molecule sequencing technologies are amenable to the methods disclosed herein.

In some embodiments, the disclosed methods comprise contacting the cross-linked double stranded nucleic acid construct ("the cross-linked construct") with an enzyme which sequentially processes the first strand and the second strand of the cross-linked construct. For example, in some embodiments the enzyme may process the first strand and then proceed to process the second strand. In some embodiments the enzyme may process the first strand, the inter-strand cross-link and the second strand. In some embodiments the enzyme may process from the first strand through the inter-strand cross-link to the second strand. In some embodiments the enzyme may sequentially process the first strand and the second strand without processing through the inter-strand cross-link. The inter-strand cross-link holds the first and second strands together so that the enzyme sequentially processes the first and second strands. Polynucleotide-processing enzymes are described in more detail below. In some embodiments, methods which comprise contacting the double stranded nucleic acid construct with an enzyme which sequentially processes the first strand and the second strand of the cross-linked construct can be used to provide additional information on the construct, such as orthogonal proof-reading sequence information.

The methods disclosed herein are advantageous as they provide additional information compared to conventional methods. As mentioned above, in conventional methods, only one strand of a double stranded nucleic acid is sequenced. If both strands are to be sequenced, it is typically necessary to use an adaptor such as a hairpin adaptor. The methods disclosed herein do not rely on using a hairpin adaptor and thus can be conducted without the downsides associated with the use of hairpin adaptors, such as increased complexity, potential for contamination and the time requirement for the adaptor to be connected to the target double stranded nucleic acid.

### Inter-Strand Cross-Links

As used herein, the term inter-strand cross-link refers to a chemical bond between the first and second strand of a double stranded nucleic acid. The chemical bond may be for example a covalent bond or an ionic bond. Typically, the term inter-strand cross-link refers to a covalent linkage between the first and second strand of a double stranded nucleic acid. The linkage may thus be between the template strand and the complement strand of a double stranded nucleic acid. The double stranded nucleic acid is typically double stranded DNA.

Typically, the inter-strand cross-link formed in the methods disclosed herein is irreversible. However, in some embodiments the inter-strand cross-link is reversibly formed. Reversible inter-strand cross-links can be exploited to reverse the linking process if desired.

In some embodiments, the inter-strand cross-link is formed between nucleobases in the first and second strands of the double stranded nucleic acid. In some embodiments, the inter-strand cross-link is formed between a nucleobase on the first strand selected from adenine (A), cytosine (C), guanine (G), and thymine (T); and a nucleobase on the second strand selected from adenine (A), cytosine (C), guanine (G), and thymine (T). In some embodiments the inter-strand cross-link is formed between a guanine residue on the first strand and a guanine residue on the second strand. In some embodiments the inter-strand cross-link is formed between a guanine residue on the first strand and a cytosine residue on the second strand or between a cytosine residue on the first strand and a guanine residue on the second strand. In some embodiments the inter-strand cross-link is formed between a thymine residue on the first strand and a thymine residue on the second strand.

In some embodiments, the inter-strand cross-link is formed between sugar groups in the first and second strands of the double stranded nucleic acid. For example, the interstrand cross-link may be formed between a deoxyribose group in the first strand and a deoxyribose group in the second strand.

In some embodiments the inter-strand cross-link is formed between nucleobases in the first and second strands of the double stranded nucleic acid and between sugar groups in the first and second strands of the double stranded nucleic acid.

In some embodiments the inter-strand cross-link is formed between a nucleobase in the first strand of the double stranded nucleic acid and a sugar group in the second strand of the double stranded nucleic acid. In some embodiments the inter-strand cross-link is formed between the backbone of the first strand of the double stranded nucleic acid and a nucleobase of the second strand of the double stranded nucleic acid. In some embodiments the inter-strand cross-link is formed between the backbone of the first strand of the double stranded nucleic acid and a sugar group of the second strand of the double stranded nucleic acid. In some embodiments the inter-strand cross-link is formed between the backbone of the first strand of the double stranded nucleic acid and the backbone of the second strand of the double stranded nucleic acid. Most often in the methods disclosed herein the interstrand cross-link is formed between nucleobases in the first and second strands of the double stranded nucleic acid.

In the methods disclosed herein, the inter-strand cross-link is formed by contacting the double stranded nucleic acid with a cross-linking agent. In some embodiments, the cross-linking agent is a cross-linking condition such as exposure to electromagnetic radiation. As used here, the term "cross-linking agent" embraces conditions which promote the formation of inter-strand cross-links such as exposure to electromagnetic radiation, such as UV light.

Any suitable cross-linking agent can be used in the methods disclosed herein. For example, in some embodiments the cross-linking agent is or comprises electromagnetic radiation (e.g. the cross-linking agent is an optical cross-linking agent). In some embodiments the cross-linking agent is or comprises a chemical reagent. Many suitable chemical reagents are known to those skilled in the art. In some embodiments the chemical reagent is a polynucleotide-interacting molecule. In some embodiments the cross-linking agent is or comprises a polynucleotide-interacting enzyme. In some embodiments the cross-linking agent is or comprises a nucleic acid cross-linking enzyme. The disclosed methods may comprise the use of multiple such cross-linking agents, e.g. a combination of such cross-linking agents can be used. For example, in some embodiments the formation of the inter-strand cross-link comprises the use of electromagnetic radiation and a chemical reagent. In some embodiments the formation of the inter-strand cross-link comprises the use of both a chemical reagent and a nucleic acid cross-linking enzyme.

In some embodiments, the cross-linking agent is chosen or determined to promote the formation of inter-strand cross-links rather than unwanted reactions (e.g. strand scission or cleavage, base damage, intra-strand cross-links, etc). In some embodiments, control of the position and/or extent of inter-strand cross-linking is achieved by using suitable cross-linking reagents and/or by controlling the reaction conditions, e.g. the concentration of the inter-strand cross-linking agent used.

In some embodiments the cross-linking agent is or comprises a clastogenic chemical reagent. In some embodiments the cross-linking agent is a chemical reagent which is a bifunctional reactive group.

In some embodiments the chemical reagent is a bifunctional alkylating agent. Bifunctional alkylating agents include nitrogen mustards and lipid peroxidation products.

Nitrogen mustards are bifunctional alkylating agents. Nitrogen mustards typically contain a reactive N,N-bis(2-chloroethyl)amine functional group with a variable R group. Nitrogen mustards typically react with the N7 position of guanine.

Non-limiting examples of nitrogen mustards include chlorambucil, mechlorethamine, and phosphamide mustards. Some nitrogen mustards are shown below: 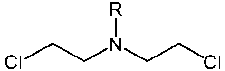 (wherein R = alkyl, alkenyl, alkynyl, aryl, aralalkyl, heteroaryl, heteroaralalkyl, phosphate etc); e.g.

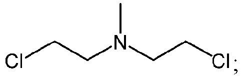

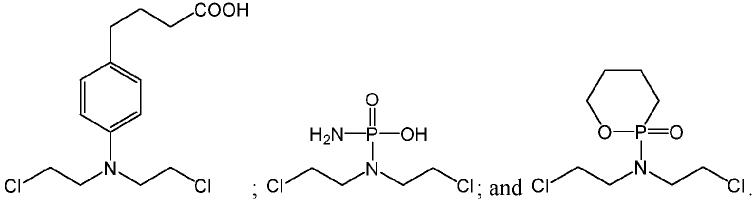

In some embodiments, the chemical reagent is a nitrogen mustard and the interstrand cross-link is formed between guanine residues on the first and second strands of the double stranded nucleic acid. In some embodiments the guanine residues are comprised in 5'-GNC-3' sequences. In some embodiments the formation of such an inter-strand crosslink leads to distortion of the helix formed by the double stranded nucleic acid in the region of the cross-link.

In some embodiments the chemical reagent is mitomycin C or an analog thereof. Mitomycin C is a bifunctional aalkylating agent. Mitomycin C is shown below:

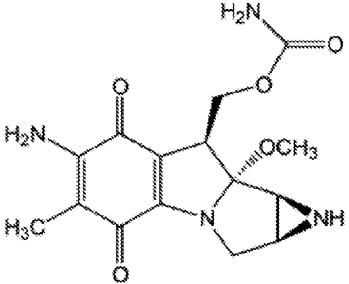

In some embodiments, the chemical agent is mitomycin C or an analog thereof and the inter-strand cross-link is formed between guanine residues on the first and second strands of the double stranded nucleic acid. In some embodiments the guanine residues are comprised in 5'-CG-3' sequences. In some embodiments the formation of such an interstrand cross-link leads to distortion of the helix formed by the double stranded nucleic acid in the region of the cross-link.

In some embodiments the chemical reagent is a lipid peroxidation product (typically an aldehyde). Non-limiting examples of lipid peroxidation products include acrolein, crotonaldehyde, and malondialdehyde. Other aldehydes which can be used include trans-4-hydroxynonenal, acetaldehyde, and formaldehyde. In some embodiments the chemical reagent is a lipid peroxidation product or other aldehyde and the inter-strand cross-link is formed between guanine residues on the first and second strands of the double stranded nucleic acid. In some embodiments the guanine residues are comprised in 5'-CG-3' or 5'-GC-3' sequences. In some embodiments the formation of such an inter-strand cross-link leads to distortion of the helix formed by the double stranded nucleic acid in the region of the cross-link.

In some embodiments the chemical reagent is a platinum compound. In some embodiments the platinum compound is cisplatin or a derivative thereof. In some embodiments the chemical reagent is cis-diamminedichloroplatinum. In some embodiments the chemical reagent is cis-diamminedichloroplatinum and the inter-strand cross-link is formed between guanine residues on the first and second strands of the double stranded nucleic acid. In some embodiments the guanine residues are comprised in 5'-GC-3' sequences. In some embodiments the formation of such an inter-strand cross-link leads to distortion of the helix formed by the double stranded nucleic acid in the region of the cross-link. In some embodiments the chemical reagent is *trans-*diamminedichloroplatinum. In some embodiments the chemical reagent is *trans-*diamminedichloroplatinum and the inter-strand cross-link is formed between a guanine residue on the first strand and a cytosine residue on the second strand of the double stranded nucleic acid. Typically prior to formation of the inter-strand cross-link the guanine residue on the first strand and the cytosine residue on the second strand are base paired. In some embodiments the formation of an inter-strand cross-link using a platinum compound leads to distortion of the helix formed by the double stranded nucleic acid in the region of the cross-link.

In some embodiments the chemical reagent is a chloro-ethyl nitrosourea such as carmustine or an analog thereof. Carmustine is shown below:

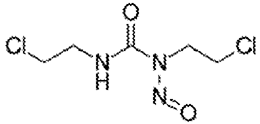

Carmustine typically react by alkylating the O⁶ position of guanine groups to form O⁶-ethanoguanine, which then crosslinks to base-paired cytosine. Thus, in some embodiments the chemical reagent is carmustine or an analog thereof and the inter-strand cross-link is formed between a guanine residue on the first strand and a cytosine residue on the second strand of the double stranded nucleic acid. Typically prior to formation of the inter-strand cross-link the guanine residue on the first strand and the cytosine residue on the second strand are base paired.

In some embodiments the chemical reagent is psoralen or a derivative thereof such as methoxypsoralen. Psoralen and methoxypsoralen are shown below:

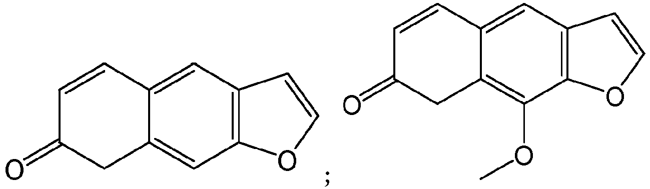

Psoralens typically intercalate into DNA and may form covalent inter-strand crosslinks e.g. when activated in the presence of light, e.g. in under Ultra Violet-A (UV-A) radiation. Such adducts are typically formed by linking the 3, 4 (pyrone) or 4', 5' (furan) edge of the psoralen to the 5, 6 double bond of thymine. Thus, in some embodiments the chemical reagent is psoralen or a derivative thereof and the inter-strand cross-link is formed between thymine residues on the first and second strands of the double stranded nucleic acid. In some embodiments the thymine residues are comprised in 5'-AT-3' sequences. In some embodiments the crosslinking agent comprises both a psoralen and electromagnetic radiation e.g. UV radiation e.g. UV-A radiation. In some embodiments the formation of an inter-strand cross-link using a psoralen leads to distortion of the helix formed by the double stranded nucleic acid in the region of the cross-link.

In some embodiments the chemical reagent is nitrous acid. Nitrous acid can form inter-strand cross-links in double stranded nucleic acid through the conversion of amino groups in DNA to carbonyls. In some embodiments the chemical reagent is nitrous acid and the inter-strand cross-link is formed between guanosines. In some embodiments the guanosine residues are comprised in 5'-CG-3' sequences. In some embodiments the formation of an inter-strand cross-link using nitrous acid leads to distortion of the helix formed by the double stranded nucleic acid in the region of the cross-link.

In some embodiments the chemical reagent is an intercalating dye. Intercalating dyes are well known in the art.

In some embodiments the chemical reagent is a reagent for promoting the formation of free radicals. Such chemical reagents include metals which react with peroxides to form or promote free radical inter-strand cross-linking.

In some embodiments the cross-linking agent comprises reagents which react with both the first and second strands on the double stranded nucleic acid to enable the reaction products to further react, thus creating an inter-strand cross-link. Such reagents are known in the art.

In some embodiments, the chemical reagent is a click chemical reagent. The reagent may promote or take part in a click chemistry reaction between nucleobases on the first and second strands of the double stranded nucleic acid. The click chemistry reaction may form an inter-strand cross-link between pendant groups on nucleobases of the first and second strands of the double stranded nucleic acid. Suitable pendant groups can be introduced into the first and/or second strands of the double stranded nucleic acid by any suitable means, for example by polymerase incorporation or by using one or more nucleic acid-modifying enzymes. For example, methyltransferases can be used to modify nucleic acids (e.g. DNA) with reactive groups, which can then link together to form inter-strand cross-links. Thus, the inter-strand cross-link may form between nucleobase adducts. Accordingly, some embodiments comprise forming the inter-strand cross-link between nucleobase adducts in the first and second strands of the target double stranded nucleic acid.

Many suitable click chemistry reagents are known in the art. Suitable examples of click chemistry include, but are not limited to, the following:
(a) copper(I)-catalyzed azide-alkyne cycloadditions (azide alkyne Huisgen cycloadditions);
(b) strain-promoted azide-alkyne cycloadditions; including alkene and azide [3+2] cycloadditions; alkene and tetrazine inverse-demand Diels-Alder reactions; and alkene and tetrazole photoclick reactions;
(c) copper-free variant of the 1,3 dipolar cycloaddition reaction, where an azide reacts with an alkyne under strain, for example in a cyclooctane ring;
(d) the reaction of an oxygen nucleophile on one linker with an epoxide or aziridine reactive moiety on the other; and
(e) the Staudinger ligation, where the alkyne moiety can be replaced by an aryl phosphine, resulting in a specific reaction with the azide to give an amide bond.

Any reactive group may be used to form the inter-strand cross-link. Some suitable reactive groups include [1, 4-Bis[3-(2-pyridyldithio)propionamido]butane; 1,1 1-bis-maleimidotriethyleneglycol; 3,3'-dithiodipropionic acid di(N-hydroxysuccinimide ester); ethylene glycol-bis(succinic acid N-hydroxysuccinimide ester); 4,4'-diisothiocyanatostilbene-2,2'-disulfonic acid disodium salt; Bis[2-(4-azidosalicylamido)ethyl] disulphide; 3-(2-pyridyldithio)propionic acid N-hydroxysuccinimide ester; 4-maleimidobutyric acid N-hydroxysuccinimide ester; Iodoacetic acid N-hydroxysuccinimide ester; S-acetylthioglycolic acid N-hydroxysuccinimide ester; azide-PEG-maleimide; and alkyne-PEG-maleimide. The reactive group may be any of those disclosed in WO 2010/086602, particularly in Table 3 of that application.

In some embodiments the cross-linking agent is or comprises electromagnetic radiation. In some embodiments the electromagnetic radiation is light radiation. In some embodiments the light radiation is visible or UV (ultraviolet) radiation. In some embodiments the radiation has a wavelength of from about 100 nm to about 600 nm, more typically from about 200 nm to about 500 nm, e.g. from about 300 nm to about 340 nm, such as from about 340 nm to about 380 nm, e.g. from about 360 to about 370 nm, e.g. about 365 nm. In some embodiments the light radiation is UV radiation. In some embodiments the UV radiation is UVA radiation. In some embodiments the UV radiation is provided by a UV laser (e.g. a gas laser, laser diode or solid state laser). In some embodiments the UV radiation is provided using a UV lamp. In some embodiments the UV radiation is high intensity UV radiation.

In some embodiments the light radiation is applied to the double stranded nucleic acid sample for between about 1 s and about 1 hour, such as between about 1 minute and about 20 minutes, e.g. from about 5 minutes to about 10 minutes, for example about 8 minutes. Those skilled in the art will be readily able to select an appropriate exposure time based on the sample being treated and the extent of inter-strand cross-links required.

As will be apparent from the above discuss, in some embodiments the cross-linking agent may comprise both a chemical reagent and electromagnetic radiation. For example, as mentioned above the combination of UV radiation and psoralens can be used to generate inter-strand cross-links. In some embodiments cross-linking the double stranded nucleic acid using an optical cross-linking agent (i.e. electromagnetic radiation as described above) may comprise the use of mediating agents, such as photosensitizers and/or radical sources.

In some embodiments the cross-linking agent is a nucleic acid cross-linking enzyme. Any suitable enzyme capable of forming an inter-strand cross-link between the first and second strands of a double stranded nucleic acid can be used.

Suitable enzymes are commercially available. For example, nucleic acid modifying enzymes including such as nucleic acid cross-linking enzymes are available from New England Biolabs (NEB, USA). For example, in some embodiments the nucleic acid cross linking enzyme is a protelomerase. In some embodiments the protelomerase is TelN (NEB catalog number M0651S). TelN Protelomerase, from phage N15, cuts dsDNA at a TelN recognition sequence (56 bp) and leaves covalently closed ends at the site of cleavage.

Nucleic acid cross-linking enzymes may also function by catalysing the formation of inter-strand cross-links using chemical reagents. For example, the DNA interstrand crosslinking agent, 5-(aziridin-1-yl)-4-hydroxylamino-2-nitrobenzamide, can be formed from 5-(aziridin-1-yl)-2,4-dinitrobenzamide (CB 1954) by a nitroreductase enzyme.

### Optionally allowing the double stranded nucleic acid strand to break

As discussed, the methods disclosed herein comprise forming an inter-strand crosslink between the first and second strands of a double stranded nucleic acid. In some embodiments, the methods may further comprise allowing the double stranded nucleic acid to break at or in the vicinity of the inter-strand cross-link. Allowing the double stranded nucleic acid to break at or in the vicinity of the inter-strand cross-link forms at least one double-stranded nucleic acid construct cross-linked at or in the vicinity of the terminus of the construct.

In some embodiments allowing the double stranded nucleic acid to break forms one double stranded nucleic acid construct. In some embodiments allowing the double stranded nucleic acid to break forms two double stranded nucleic acid constructs.

For example, in some embodiments forming an inter-strand cross-link comprises forming one bond (inter-strand cross-link) between the first and second strands of the double stranded nucleic acid. Breaking the double stranded nucleic acid at or in the vicinity of the inter-strand cross-link thus forms a portion of the double stranded nucleic acid which comprises the inter-strand cross-link between the first and second strands, and a portion wherein the first and second strands are not linked by an inter-strand cross-link. The portion which comprises the inter-strand cross-link between the first and second strands corresponds to the cross-linked double-stranded nucleic acid construct. The portion which does not comprise the inter-strand cross-link between the first and second strands may remain as a double stranded nucleic acid or may be dissociate into single stranded nucleic acids.

In some embodiments forming an inter-strand cross-link comprises forming two or more bonds (inter-strand cross-links) between the first and second strands of the double stranded nucleic acid.

In some embodiments breaking the double stranded nucleic acid at or in the vicinity of the inter-strand cross-links comprises breaking the double stranded nucleic acid between two of the two or more inter-strand cross-links, thus forming two portions of double stranded nucleic acid each comprising an inter-strand cross-link between the first and second strands. Either or both of the portions of the double stranded nucleic acid comprising an inter-strand cross-link between the first and second strands correspond to the cross-linked double-stranded nucleic acid construct.

In some embodiments breaking the double stranded nucleic acid at or in the vicinity of the inter-strand cross-links forms a portion of the double stranded nucleic acid which comprises two or more bonds (inter-strand cross-links) between the first and second strands, and a portion wherein the first and second strands are not linked by an inter-strand cross-link. The portion which comprises the inter-strand cross-links between the first and second strands corresponds to the cross-linked double-stranded nucleic acid construct . The portion which does not comprise inter-strand cross-links between the first and second strands may remain as a double stranded nucleic acid or may be dissociate into single stranded nucleic acids.

In embodiments which comprise forming a plurality of inter-strand cross-links and forming an inter-strand cross-linked portion comprising at least two inter-strand crosslinks, the construct thereby formed can be used to prepare a repeating circular synthesis product. In some embodiments, melting and invading primers can be used to provide an initiation point for polymerase mediated strand copying. In some embodiments a polynucleotide-processing enzyme, e.g. a polymerase cannot proceed through the interstrand cross-link sites whilst remaining on the same strand, so when encountering the interstrand cross-links the polymerase switches to the adjacent strand. In this way, the synthesized strand thus becomes complementary to the adjacent strand. The polynucleotide-processing enzyme (e.g. the polymerase) may then proceed to replicate around the "circular" region formed between the inter-strand cross-links. This process is shown schematically in Figure 4. Those skilled in the art will appreciate that in such embodiments, formation of a repeating circular synthesis product does not require that the double stranded nucleic acid is allowed to break. However it is not excluded that the double stranded nucleic acid is allowed to break outside the portion which is bounded by the two inter-strand cross-links. Thus, such embodiments are amenable to methods which comprise allowing the double stranded nucleic acid to break, and to those embodiments which do not comprise allowing the double stranded nucleic acid to break.

In some embodiments breaking the double stranded nucleic acid comprises breaking both strands of the double stranded nucleic acid. In some embodiments breaking the double stranded nucleic acid comprises breaking one strand of the double stranded nucleic acid.

By way of non-limiting example, some of these embodiments are illustrated schematically in Figure 1. Fig. 1A shows the formation of an inter-strand cross-link between first and second strands of a double stranded nucleic acid followed by a break in both strands of the double stranded nucleic acid in the vicinity of the inter-strand cross-link to form a double-stranded nucleic acid construct cross-linked in the vicinity of the terminus of the construct and a non-cross-linked portion. Fig. 1B shows the formation of an interstrand cross-link between first and second strands of a double stranded nucleic acid followed by a break in one strand of the double stranded nucleic acid in the vicinity of the inter-strand cross-link to form a double-stranded nucleic acid construct cross-linked in the vicinity of the terminus of the construct and a non-cross-linked (single-stranded) portion. Fig. 1C shows the formation of a multiplicity of inter-strand cross-links between first and second strands of a double stranded nucleic acid (two cross-links of the multiplicity of cross-links are shown) followed by a break in both strands of the double stranded nucleic acid between two of the multiplicity of cross-links to form two double-stranded nucleic acid constructs each cross-linked in the vicinity of the terminus of the construct. Fig. 1D shows the formation of a multiplicity of inter-strand cross-links between first and second strands of a double stranded nucleic acid (two cross-links of the multiplicity of cross-links are shown) followed by a break in both strands of the double stranded nucleic acid, to form a double-stranded nucleic acid construct cross-linked in the vicinity of the terminus of the construct and a non-cross-linked portion.

As explained above, some embodiments of the methods disclosed herein comprise allowing the target double stranded nucleic acid to break at or in the vicinity of the interstrand cross-link. In some embodiments the methods comprise breaking the target double stranded nucleic acid at or in the vicinity of the inter-strand cross-link. In some embodiments the method comprise inducing the target double stranded nucleic acid to break at or in the vicinity of the inter-strand cross-link.

In some embodiments the disclosed methods comprise allowing the target double stranded nucleic acid to break within about 500 nucleotides of the inter-strand cross-link. In some embodiments the disclosed methods comprise allowing the target double stranded nucleic acid to break within between about 1 nucleotide and about 200 nucleotides of the inter-strand cross-link. In some embodiments the method comprises allowing the target double stranded nucleic acid to break within between about 5 nucleotide and about 150 nucleotides, e.g. between about 10 nucleotides and about 100 nucleotides, such as between about 20 nucleotides and about 80 nucleotides, e.g. between about 30 nucleotides and about 50 nucleotides of the inter-strand cross-link. In such embodiments, allowing the double stranded nucleic acid to break at or in the vicinity of the inter-strand cross-link forms an "overhang region" adjacent to the inter-strand cross-link. The length of the overhang region corresponds to the number of nucleotides between the inter-strand crosslink and the break in the double stranded nucleic acid.

Accordingly, in some embodiments the cross-link formed in the double stranded nucleic acid is positioned within about 500 nucleotides of the terminus of the cross-linked construct formed by contacting the double stranded nucleic acid with the cross-linking agent and subsequently allowing it to break. In some embodiments the cross-link is positioned within from about 1 nucleotide and about 200 nucleotides of terminus thus formed. In some embodiments the cross-link is positioned within between about 5 nucleotide and about 150 nucleotides, e.g. between about 10 nucleotides and about 100 nucleotides, such as between about 20 nucleotides and about 80 nucleotides, e.g. between about 30 nucleotides and about 50 nucleotides of the terminus of the cross-linked construct formed by contacting the double stranded nucleic acid with the cross-linking agent and subsequently allowing it to break.

In some embodiments the methods comprise breaking the double stranded nucleic acid at the position of the inter-strand cross-link so that no overhang region forms.

Some embodiments comprise forming the inter-strand cross-link at or in the vicinity of the terminus of the target double stranded nucleic acid. In such embodiments, it may not be necessary to allow the double stranded nucleic acid to break. Thus, in some embodiments the cross-link formed in the target double stranded nucleic acid is positioned within about 500 nucleotides of the terminus of the target double stranded nucleic acid. In some embodiments the cross-link is positioned within from about 1 nucleotide and about 200 nucleotides of the terminus of the target double stranded nucleic acid. In some embodiments the cross-link is positioned within between about 5 nucleotide and about 150 nucleotides, e.g. between about 10 nucleotides and about 100 nucleotides, such as between about 20 nucleotides and about 80 nucleotides, e.g. between about 30 nucleotides and about 50 nucleotides of the terminus of the target double stranded nucleic acid. In other words, in some embodiments the terminus of the target double stranded nucleic acid is within about 500 nucleotides of the inter-strand cross-link formed in the disclosed methods. In some embodiments the terminus of the target double stranded nucleic acid is within from about 1 nucleotide and about 200 nucleotides of the inter-strand cross-link formed in the disclosed methods. In some embodiments the terminus of the target double stranded nucleic acid is within between about 5 nucleotide and about 150 nucleotides, e.g. between about 10 nucleotides and about 100 nucleotides, such as between about 20 nucleotides and about 80 nucleotides, e.g. between about 30 nucleotides and about 50 nucleotides of the inter-strand cross-link formed in the disclosed methods.

In embodiments of the disclosed methods which comprise allowing the double stranded nucleic acid to break, any suitable method can be used to break the double stranded nucleic acid. In some embodiments the break occurs naturally. In some embodiments the break occurs naturally due to distortion. In some embodiments the distortion is formed by the inter-strand cross-link. Non-limiting examples of reagents which produce inter-strand cross-links which distort the structure of the double stranded nucleic acid are provided above. Accordingly, in some embodiments, allowing the double stranded nucleic acid to break comprises allowing the double stranded nucleic acid to break due to distortion in the double stranded nucleic acid. In some embodiments the distortion is induced by the inter-strand cross-link.

In some embodiments the break is induced by the cross-linking reaction. For example, in some embodiments the cross-linking reaction forms an inter-strand cross-link between the backbone of the first strand and the backbone of the second strand of the target double stranded nucleic acid and simultaneously causes the double stranded nucleic acid to break.

In some embodiments the break is induced by physical agitation. Accordingly, in some embodiments allowing the double stranded nucleic acid to break comprises physically agitating the double stranded nucleic acid. Any suitable form of physical agitation can be used. For example, the break can be induced by pipetting, vortexing, sonicating, stirring, acoustic shearing, nebulization, point-sink shearing, etc.

In some embodiments the break is induced by a chemical or biological reagent. For example, in some embodiments allowing the double stranded nucleic acid to break comprises contacting the double stranded nucleic acid with a chemical or biological reagent. In some embodiments the biological reagent is an enzyme. In some embodiments the enzyme is an enzyme involved in DNA repair *in vivo.* Repair enzymes are known to detect damage and cleave double stranded nucleic acids at inter-strand cross-links. Natural or engineered enzymes can be used to cleave at or in the vicinity of inter-strand cross-links while leaving the inter-strand cross-link intact. Suitable reagents include e.g. restriction enzymes. Suitable restriction enzymes are commercially available, e.g. from New England Biolabs (USA). Some suitable restriction enzymes are listed at https://international.neb.com/products/restriction-endonucleases.

In some embodiments the inter-strand cross-linked construct formed in the disclosed methods is treated with one or more enzymes e.g. one or more exonucleases after the crosslinking process to degrade the exposed ends of the double stranded polynucleotide. In some such embodiments, the method does not comprise allowing the double stranded nucleic acid to break. In some such embodiments, the method also comprises allowing the double stranded nucleic acid to break.

Non-limiting examples of exonucleases which can be used to specifically degrade the ends of dsDNA until encountering the cross-link include exonuclease III from *E. coli*; T7 exonuclease; exonuclease V (RecBCD); exonuclease VIII, truncated; and Lambda exonuclease.

In some embodiments treatment of the cross-linked double stranded nucleic acid construct using one or more exonucleases leaves little or no overhanging strand. In embodiments in which an overhanging strand is left, this can be removed using a suitable enzyme, e.g. Mung Bean Endonuclease.

In some embodiments, the inter-strand cross-linked construct may be further processed prior to being sequenced using a single molecule sequencing technique. For example, in some embodiments the inter-strand cross-linked construct may be end-repaired if required, e.g. using the NEBNext^{(R)} End Repair module from New England Biolabs or equivalent reagents. In some embodiments the inter-strand cross-linked construct may have one or more tails ligated thereto, e.g. a dA tail may be ligated to the construct, for example by using the NEBNext^{(R)} dA-tailing module from New England Biolabs or equivalent reagents.

### Single Molecule Sequencing

The methods disclosed herein comprise sequencing the cross-linked construct formed by cross-linking the first and second strands of the double stranded nucleic acid using a single molecule sequencing technique.

The methods disclosed herein are amenable to use with any suitable technique. As explained in more detail herein, in some embodiments the methods provide orthogonal proof-reading sequence information. As such, they are suitable for use with those single-molecule techniques for which orthogonal proof-reading sequence information is useful. Suitable single molecule sequencing techniques include nanopore strand sequencing (i.e. sequencing using a nanopore sensor), and sequencing by synthesis. These techniques are described in more detail herein.

In nanopore strand sequencing, the target analyte (i.e. the cross-linked construct) moves with respect to, such as into or through, a transmembrane nanopore as described in more detail herein. The signal recorded as the cross-linked construct moves with respect to the nanopore allows the sequence of the target double stranded nucleic acid to be determined. Other characteristics of the construct, for example (i) the length of the polynucleotide, (ii) the identity of the polynucleotide, (iii) the secondary structure of the polynucleotide and (iv) whether or not the polynucleotide is modified, can also be determined.

In some embodiments of nanopore strand sequencing, the binding of a molecule (e.g. a target polynucleotide) in the channel of a nanopore has an effect on the open-channel ion flow through the pore. This is the essence of "molecular sensing" of pore channels. Variation in the open-channel ion flow can be measured using suitable measurement techniques, e.g. by the change in electrical current (for example, WO 2000/28312 and D. Stoddart et al., Proc. Natl. Acad. Sci., 2010, 106, 7702-7 or WO 2009/077734). The degree of reduction in ion flow, as measured by the reduction in electrical current, is related to the size of the obstruction within, or in the vicinity of, the pore. Analogous information can be obtained using optical methods, for example as disclosed in Huang et al., Nature Nanotechnology 10, 986-991(2015). Binding of a molecule of interest (e.g. the target polynucleotide) in or near the pore therefore provides a detectable and measurable event, thereby forming the basis of a "biological sensor".

As a nucleic acid molecule, or an individual base, moves with respect to a nanopore (e.g. as it passes through the channel of a nanopore), the size differential between the bases causes a directly correlated reduction in the ion flow through the channel. The variation in ion flow may be recorded. Suitable electrical measurement techniques for recording ion flow variations are described in, for example, WO 2000/28312 and D. Stoddart et al., Proc. Natl. Acad. Sci., 2010, 106, pp 7702-7 (single channel recording equipment); and, for example, in WO 2009/077734 (multi-channel recording techniques). Through suitable calibration, the characteristic reduction in ion flow can be used to identify the particular nucleotide and associated base traversing the channel in real-time. In typical nanopore nucleic acid sequencing, the open-channel ion flow is reduced as the individual nucleotides of the nucleic sequence of interest sequentially pass through the channel of the nanopore due to the partial blockage of the channel by the nucleotide. It is this reduction in ion flow that is measured using the suitable recording techniques described above. The reduction in ion flow may be calibrated to the reduction in measured ion flow for known nucleotides through the channel resulting in a means for determining which nucleotide is passing through the channel, and therefore, when done sequentially, a way of determining the nucleotide sequence of the nucleic acid passing through the nanopore. For the accurate determination of individual nucleotides, it has typically required for the reduction in ion flow through the channel to be directly correlated to the size of the individual nucleotide passing through the constriction (or "reading head"). It will be appreciated that sequencing may be performed upon an intact nucleic acid polymer that is 'threaded' through the pore via the action of an associated motor protein such as a polymerase or helicase, for example. Suitable motor proteins are described in more detail herein. Alternatively, sequences may be determined by passage of nucleotide triphosphate bases that have been sequentially removed from a target nucleic acid in proximity to the pore (see for example WO 2014/187924).

In other embodiments, the sequence of the construct is determined using sequencing by synthesis. An example of sequencing by synthesis is single-molecule real-time sequencing.

Single-molecule real-time sequencing is a parallelized single molecule DNA sequencing method. In embodiments of the disclosed methods in which the sequence of the double stranded nucleic acid construct is determined using single-molecule real-time sequencing, a nucleic acid processing enzyme such as a DNA polymerase is typically constrained in a zero-mode waveguide. The zero-mode waveguide may for example comprise a nanophotonic confinement structure comprising a hole of typical size ca. 70 nm in diameter, ca. 100 nm depth, in an cladding film, e.g. an aluminium cladding film, deposited on a substrate such as silica. Suitable enzymes, e.g. suitable polymerases, are described in more detail herein.

A single strand of the double stranded nucleic acid construct is contacted with the polymerase. The polymerase catalyses the incorporation of labelled nucleotides, e.g. optically (e.g. fluorescently) labelled nucleotides, to synthesize a new strand complementary to the construct strand as it is processed by the polymerase. Incorporation of the labelled nucleotides generates a detectable signal characteristic of the nucleotide being incorporated, and thus complementary to the nucleotide at that position in the template (construct) strand. A plurality of zero-mode waveguides may be used in an array in order to provide improved data throughput.

### Sequential Processing and Proof-reading

The methods disclosed herein typically allow for improved information to be obtained as a single-molecule sequencing technique is used to sequence the double stranded nucleic acid construct. For example, in some embodiments, by contacting the cross-linked construct with an enzyme which sequentially processes the first strand, optionally the cross-link, and the second strand of the construct, orthogonal proof-reading sequence information is provided.

Those skilled in the art will appreciate that proof-reading sequence information can be obtained using the methods disclosed herein because if both strands of the double stranded nucleic acid construct, linked by the inter-strand cross-link, are sequenced, then each position in the double stranded nucleic acid is not merely sequenced once, but is interrogated twice. In other words, if the strands of the double stranded nucleic acid are not linked together e.g. by an inter-strand cross-link, then conventional techniques typically only sequence one strand of the double stranded nucleic acid. The other strand is typically discarded. However, in embodiments of the disclosed methods in which both strands of the double stranded nucleic acid construct are sequentially processed because they are attached together by the inter-strand cross-link, each base pair is probed twice, once as the template strand is sequenced and then again as the linked complement strand is sequenced. This "dual" sequencing of both template and complement strand, attached together by the inter-strand cross-link, provides orthogonal sequence information. The information is orthogonal because a first nucleotide in the first strand is complementary to the corresponding nucleotide in the second strand. For example, when a C base is sequenced in the first strand, the corresponding position in the second strand will be a G base. Base calling algorithms can exploit this relationship to use the information obtained as the second strand of the construct is sequenced to "proof-read" the information obtained from the first strand as it is sequenced.

This ability to interrogate each position twice is particularly important when sequencing nucleic acids using stochastic sensing such as nanopore sensing. Such sequencing normally depends on the detection of every base in turn, e.g. by its capture by a transmembrane nanopore, and a sufficiently high sampling rate is required to enable accurate determination of the captured bases. Being able to effectively interrogate every base twice reduces the need to capture every base at a sufficiently high rate.

The ability to interrogate each position twice is also helpful for differentiating between similar bases, for example by distinguishing between methylcytosine and thymine. These two bases can sometimes result in similar signals when they are characterized by stochastic sequencing e.g. using a transmembrane pore. It can therefore be difficult to differentiate between the two. However, interrogation of each position in a nucleic acid twice will allow such differentiation because the complementary base for methylcytosine is guanine, whereas the complementary base for thymine is adenine. Methylcytosine has been linked with various diseases, including cancer.

### Target double stranded nucleic acid

As explained in more detail herein, the methods provided herein are methods of sequencing a target double stranded nucleic acid. A double stranded nucleic acid can also be referred to as a double stranded polynucleotide.

In some embodiments the double stranded nucleic acid is secreted from cells. Alternatively, the double stranded nucleic acid can be an analyte that is present inside cells such that it must be extracted from cells for sequencing.

The double stranded nucleic acid to be characterised in the methods described herein may be provided as an impure mixture of one or more target analytes and one or more impurities. Impurities may comprise truncated forms of target polynucleotide analytes which are distinct from the target analytes. For example the target analyte may be genomic DNA and impurities may comprise fractions of genomic DNA, plasmids, etc. The target polynucleotide may be a coding region of genomic DNA and undesired polynucleotides may comprise non-coding regions of DNA.

Examples of polynucleotides include DNA and RNA. The bases in DNA and RNA may be distinguished by their physical size.

A polynucleotide or nucleic acid may comprise any combination of any nucleotides. The nucleotides can be naturally occurring or artificial. One or more nucleotides in the polynucleotide can be oxidized or methylated. One or more nucleotides in the polynucleotide may be damaged. For instance, the polynucleotide may comprise a pyrimidine dimer. Such dimers are typically associated with damage by ultraviolet light and are the primary cause of skin melanomas.

One or more nucleotides in the polynucleotide may be modified, for instance with a label or a tag, for which suitable examples are known by a skilled person. The polynucleotide may comprise one or more spacers. An adapter, for example a sequencing adapter, may be comprised in the polynucleotide. Adapters, tags and spacers are described in more detail herein.

Examples of modified bases are disclosed herein and can be incorporated into the target double stranded nucleic acid by means known in the art, e.g. by polymerase incorporation of modified nucleotide triphosphates during strand copying (e.g. in PCR) or by polymerase fill-in methods. In some embodiments one or more bases can be modified by chemical means using reagents known in the art.

A nucleotide typically contains a nucleobase, a sugar and at least one phosphate group. The nucleobase and sugar form a nucleoside. The nucleobase is typically heterocyclic. Nucleobases include, but are not limited to, purines and pyrimidines and more specifically adenine (A), guanine (G), thymine (T), uracil (U) and cytosine (C). The sugar is typically a pentose sugar. Nucleotide sugars include, but are not limited to, ribose and deoxyribose. The sugar is preferably a deoxyribose. The polynucleotide preferably comprises the following nucleosides: deoxyadenosine (dA), deoxyuridine (dU) and/or thymidine (dT), deoxyguanosine (dG) and deoxycytidine (dC). The nucleotide is typically a ribonucleotide or deoxyribonucleotide. The nucleotide typically contains a monophosphate, diphosphate or triphosphate. The nucleotide may comprise more than three phosphates, such as 4 or 5 phosphates. Phosphates may be attached on the 5' or 3' side of a nucleotide. The nucleotides in the polynucleotide may be attached to each other in any manner. The nucleotides are typically attached by their sugar and phosphate groups as in nucleic acids. The nucleotides may be connected via their nucleobases as in pyrimidine dimers.

The target polynucleotide is double stranded. The target polynucleotide may be double stranded DNA. The target polynucleotide may be double stranded RNA. The target polynucleotide may be an DNA-RNA hybrid. DNA-RNA hybrids can be prepared from single-stranded RNA by reverse transcribing the cDNA complement.

The polynucleotide is most preferably double stranded deoxyribonucleic acid (DNA) or double stranded ribonucleic nucleic acid (RNA).

In addition to determining the sequence of the double stranded nucleic acid, the methods disclosed herein may comprise determining one or more other characteristics of the double stranded nucleic acid selected from (i) the length of the polynucleotide, (ii) the identity of the polynucleotide, (iii) the secondary structure of the polynucleotide and (iv) whether or not the polynucleotide is modified.

The polynucleotide can be any length (i). For example, the polynucleotide can be at least 10, at least 50, at least 100, at least 150, at least 200, at least 250, at least 300, at least 400 or at least 500 nucleotides or nucleotide pairs in length. The polynucleotide can be 1000 or more nucleotides or nucleotide pairs, 5000 or more nucleotides or nucleotide pairs in length or 100000 or more nucleotides or nucleotide pairs in length. Any number of polynucleotides can be investigated. For instance, the method may concern characterising 2, 3, 4, 5, 6, 7, 8, 9, 10, 20, 30, 50, 100 or more polynucleotides. If two or more polynucleotides are characterised, they may be different polynucleotides or two instances of the same polynucleotide. The polynucleotide can be naturally occurring or artificial. For instance, the method may be used to verify the sequence of a manufactured oligonucleotide. The method is typically carried out *in vitro.*

Nucleotides can have any identity (ii), and include, but are not limited to, adenosine monophosphate (AMP), guanosine monophosphate (GMP), thymidine monophosphate (TMP), uridine monophosphate (UMP), 5-methylcytidine monophosphate, 5-hydroxymethylcytidine monophosphate, cytidine monophosphate (CMP), cyclic adenosine monophosphate (cAMP), cyclic guanosine monophosphate (cGMP), deoxyadenosine monophosphate (dAMP), deoxyguanosine monophosphate (dGMP), deoxythymidine monophosphate (dTMP), deoxyuridine monophosphate (dUMP), deoxycytidine monophosphate (dCMP) and deoxymethylcytidine monophosphate. The nucleotides are preferably selected from AMP, TMP, GMP, CMP, UMP, dAMP, dTMP, dGMP, dCMP and dUMP. A nucleotide may be abasic (i.e. lack a nucleobase). A nucleotide may also lack a nucleobase and a sugar (i.e. is a C3 spacer). The sequence of the nucleotides in the double stranded nucleic acid may be determined by the consecutive identity of following nucleotides attached to each other throughout the polynucleotide strain, in the 5' to 3' direction of the strand.

The target double stranded nucleic acid may comprise the products of a PCR reaction, genomic DNA, the products of an endonuclease digestion and/or a DNA library. The target double stranded nucleic acid may may be obtained from or extracted from any organism or microorganism. The target double stranded nucleic acid may is often obtained from a human or animal, e.g. from urine, lymph, saliva, mucus, seminal fluid or amniotic fluid, or from whole blood, plasma or serum. The target double stranded nucleic acid may may be obtained from a plant e.g. a cereal, legume, fruit or vegetable. The target double stranded nucleic acid may may comprise genomic DNA. The genomic DNA may be fragmented. The DNA may be fragmented by any suitable method. For example, methods of fragmenting DNA are known in the art, Such methods may use a transposase, such as a MuA transposase. Often the genomic DNA is not fragmented. In some embodiments, the target double stranded nucleic acid may may be DNA, RNA and/or a DNA/RNA hybrid.

It is within the scope of the methods provided herein that the analyte is labelled with a molecular label. A molecular label may be a modification to the analyte which promotes the detection of the analyte in the methods provided herein. For example the label may be a modification to the analyte which alters the signal obtained as the sequence of the double stranded nucleic acid is determined. For example, in embodiments which comprise determining the sequence of the double stranded nucleic acid as the double stranded nucleic acid moves with respect to a nanopore, the label may interfere with a flux of ions through the nanopore. In such a manner, the label may improve the sensitivity of the methods.

### Adapters

In some embodiments of the methods provided herein, the double stranded nucleic acid to be sequenced has a polynucleotide adapter attached thereto. An adapter typically comprises a polynucleotide strand capable of being attached to the end of a target polynucleotide.

In some embodiments the adapter is attached to the double stranded nucleic acid before the inter-strand cross-link is formed. In some embodiments the adapter is attached to the cross-linked double stranded nucleic acid construct. In some embodiments of the disclosed methods which comprise allowing the double stranded nucleic acid to break, the adapter is attached to the double stranded nucleic acid after the inter-strand cross-link is formed but before the double stranded nucleic acid is allowed to break. In some embodiments of the disclosed methods which comprise allowing the double stranded nucleic acid to break, the adapter is attached to the crosslinked construct formed by the breaking of the inter-strand cross-linked double stranded nucleic acid.

Accordingly, in some embodiments the methods comprise attaching an adapter (e.g. an adapter as described herein) to the target double stranded nucleic acid and forming the inter-strand cross-link in the adapter. In some embodiments the adapter may be chosen or modified in order to provide a specific site for the inter-strand cross-link. For example, in some embodiments the adapter may contain components that are specifically sensitive to crosslinking conditions such that only the adapters are crosslinked at the desired time.

An adapter may be attached to just one end of the double stranded nucleic acid or construct. A polynucleotide adapter may be added to both ends of the double stranded nucleic acid or construct. Alternatively, different adapters may be added to the two ends of the double stranded nucleic acid or construct. Discussion below relating to "a double stranded polynucleotide" relates to either the target double stranded nucleic acid or to a cross-linked construct thereof.

An adapter may be added to both strands of a double stranded polynucleotide. An adapter may be added to just one strand of a polynucleotide. Methods of adding adapters to polynucleotides are known in the art. Adapters may be attached to polynucleotides, for example, by ligation, by click chemistry, by tagmentation, by topoisomerisation or by any other suitable method.

In one embodiment, the or each adapter is synthetic or artificial. Typically, the or each adapter comprises a polymer as described herein. In some embodiments, the or each adapter comprises a spacer as described herein. In some embodiments, the or each adapter comprises a polynucleotide. The or each polynucleotide adapter may comprise DNA, RNA, modified DNA (such as abasic DNA), RNA, PNA, LNA, BNA and/or PEG. Usually, the or each adapter comprises single stranded and/or double stranded DNA or RNA. The adapter may comprise the same type of polynucleotide as the polynucleotide strand to which it is attached. The adapter may comprise a different type of polynucleotide to the polynucleotide strand to which it is attached. In some embodiments the polynucleotide strand assessed and characterised in the methods described herein is a double stranded DNA strand and the adapter comprises DNA or RNA, e.g. double or single stranded DNA.

In some embodiments, an adapter may be a bridging moiety. A bridging moiety may be used to connect the two strands of a double-stranded polynucleotide. For example, in some embodiments a bridging moiety is used to connect the template strand of a double stranded polynucleotide to the complement strand of the double stranded polynucleotide.

A bridging moiety typically covalently links the two strands of the target polynucleotide. The bridging moiety can be anything that is capable of linking the two strands of the target polynucleotide, provided that the bridging moiety does not interfere with movement of the single stranded polynucleotide through the transmembrane pore. Suitable bridging moieties include, but are not limited to a polymeric linker, a chemical linker, a polynucleotide or a polypeptide. Preferably, the bridging moiety comprises DNA, RNA, modified DNA (such as abasic DNA), RNA, PNA, LNA or PEG. The bridging moiety is more preferably DNA or RNA.

In some embodiments a bridging moiety is a hairpin adapter. A hairpin adapter is an adapter comprising a single polynucleotide strand, wherein the ends of the polynucleotide strand are capable of hybridising to each other, or are hybridized to each other, and wherein the middle section of the polynucleotide forms a loop. Suitable hairpin adapters can be designed using methods known in the art. In some embodiments a hairpin loop is typically 4 to 100 nucleotides in length, e.g. from 4 to 50 such as from 4 to 20 e.g. from 4 to 8 nucleotides in length. In some embodiments the bridging moiety (e.g. hairpin adapter) is attached at one end of the target polynucleotide. A bridging moiety (e.g. hairpin adapter) is typically not attached at both ends of the target polynucleotide.

In some embodiments, an adapter is a linear adapter. A linear adapter may be bound to either or both ends of a single stranded polynucleotide. When the polynucleotide is a double stranded polynucleotide, a linear adapter may be bound to either or both ends of either or both strands of the double stranded polynucleotide. A linear adapter may comprise a leader sequence as described herein. A linear adapter may comprise a portion for hybridisation with a tag (such as a pore tag) as described herein. A linear adapter may be 10 to 150 nucleotides in length, such as from 20 to 120, e.g. 30 to 100, for example 40 to 80 such as 50 to 70 nucleotides in length. A linear adapter may be single stranded. A linear adapter may be double stranded.

In some embodiments, an adapter may be a Y adapter. A Y adapter is typically a polynucleotide adapter. A Y adapter is typically double stranded and comprises (a) at one end, a region where the two strands are hybridised together and (b), at the other end, a region where the two strands are not complementary. The non-complementary parts of the strands typically form overhangs. The presence of a non-complementary region in the Y adapter gives the adapter its Y shape since the two strands typically do not hybridise to each other unlike the double stranded portion. The two single-stranded portions of the Y adapter may be the same length, or may be different lengths. For example, one single-stranded portion of the Y adapter may be 10 to 150 nucleotides in length, such as from 20 to 120, e.g. 30 to 100, for example 40 to 80 such as 50 to 70 nucleotides in length and the other single stranded portion of the Y adapter may independently by 10 to 150 nucleotides in length, such as from 20 to 120, e.g. 30 to 100, for example 40 to 80 such as 50 to 70 nucleotides in length. The double-stranded "stem" portion of the Y adapter may be e.g. from 10 to 150 nucleotides in length, such as from 20 to 120, e.g. 30 to 100, for example 40 to 80 such as 50 to 70 nucleotides in length.

An adapter may be linked to the target polynucleotide by any suitable means known in the art. The adapter may be synthesized separately and chemically attached or enzymatically ligated to the target polynucleotide. Alternatively, the adapter may be generated in the processing of the target polynucleotide. In some embodiments, the adapter is linked to the target polynucleotide at or near one end of the target polynucleotide. In some embodiments, the adapter is linked to the target polynucleotide within 50, e.g. within 20 for example within 10 nucleotides of an end of the target polynucleotide. In some embodiments the adapter is linked to the target polynucleotide at a terminus of the target polynucleotide. When a adapter is linked to the target polynucleotide the adapter may comprise the same type of nucleotides as the target polynucleotide or may comprise different nucleotides to the target polynucleotide.

### Spacers

In some embodiments of the methods provided herein, the target double stranded nucleic acid to be sequenced, a construct formed by the cross-linking thereof, or an adapter as described herein, may comprise a spacer. For example, one or more spacers may be present in the polynucleotide adapter. For example, the polynucleotide adapter may comprise from one to about 10 spacers, e.g. from 1 to about 5 spacers, e.g. 1, 2, 3, 4 or 5 spacers. The spacer may comprise any suitable number of spacer units. A spacer may provide an energy barrier which impedes movement of a motor protein. For example, a spacer may stall a motor protein by reducing the traction of the motor protein on the polynucleotide. This may be achieved for instance by using an abasic spacer i.e. a spacer in which the bases are removed from one or more nucleotides in the polynucleotide adapter. A spacer may physically block movement of a motor protein, for instance by introducing a bulky chemical group to physically impede the movement of the motor protein.

In some embodiments, particularly embodiments in which the double stranded nucleic acid is sequenced using a nanopore, one or more spacers are included in the polynucleotide or construct or in an adapter as used in the methods claimed herein in order to provide a distinctive signal when they pass through or across the nanopore, i.e. as they move with respect to the nanopore.

In some embodiments, a spacer may comprise a linear molecule, such as a polymer. Typically, such a spacer has a different structure from the target polynucleotide. For instance, if the target polynucleotide is DNA, the or each spacer typically does not comprise DNA. In particular, if the target polynucleotide is deoxyribonucleic acid (DNA) or ribonucleic acid (RNA), the or each spacer preferably comprises peptide nucleic acid (PNA), glycerol nucleic acid (GNA), threose nucleic acid (TNA), locked nucleic acid (LNA) or a synthetic polymer with nucleotide side chains. In some embodiments, a spacer may comprise one or more nitroindoles, one or more inosines, one or more acridines, one or more 2-aminopurines, one or more 2-6-diaminopurines, one or more 5-bromo-deoxyuridines, one or more inverted thymidines (inverted dTs), one or more inverted dideoxy-thymidines (ddTs), one or more dideoxy-cytidines (ddCs), one or more 5-methylcytidines, one or more 5-hydroxymethylcytidines, one or more 2'-O-Methyl RNA bases, one or more Iso-deoxycytidines (Iso-dCs), one or more Iso-deoxyguanosines (Iso-dGs), one or more C3 (OC₃H₆OPO₃) groups, one or more photo-cleavable (PC) [OC₃H₆-C(O)NHCH₂-C₆H₃NO₂-CH(CH₃)OPO₃] groups, one or more hexandiol groups, one or more spacer 9 (iSp9) [(OCH₂CH₂)₃OPO₃] groups, or one or more spacer 18 (iSp18) [(OCH₂CH₂)₆OPO₃] groups; or one or more thiol connections. A spacer may comprise any combination of these groups. Many of these groups are commercially available from IDT^{®} (Integrated DNA Technologies^{®}). For example, C3, iSp9 and iSp18 spacers are all available from IDT^{®}. A spacer may comprise any number of the above groups as spacer units.

In some embodiments, a spacer may comprise one or more chemical groups which cause a motor protein to stall. In some embodiments, suitable chemical groups are one or more pendant chemical groups. The one or more chemical groups may be attached to one or more nucleobases in the polynucleotide, construct or adapter. The one or more chemical groups may be attached to the backbone of the polynucleotide adapter. Any number of appropriate chemical groups may be present, such as 2, 3, 4, 5, 6, 7, 8, 9, 10, 11, 12 or more. Suitable groups include, but are not limited to, fluorophores, streptavidin and/or biotin, cholesterol, methylene blue, dinitrophenols (DNPs), digoxigenin and/or antidigoxigenin and dibenzylcyclooctyne groups. In some embodiments, a spacer may comprise a polymer. In some embodiments the spacer may comprise a polymer which is a polypeptide or a polyethylene glycol (PEG).

In some embodiments, a spacer may comprise one or more abasic nucleotides (i.e. nucleotides lacking a nucleobase), such as 2, 3, 4, 5, 6, 7, 8, 9, 10, 11, 12 or more abasic nucleotides. The nucleobase can be replaced by -H (idSp) or -OH in the abasic nucleotide. Abasic spacers can be inserted into target polynucleotides by removing the nucleobases from one or more adjacent nucleotides. For instance, polynucleotides may be modified to include 3-methyladenine, 7-methylguanine, 1,N6-ethenoadenine inosine or hypoxanthine and the nucleobases may be removed from these nucleotides using Human Alkyladenine DNA Glycosylase (hAAG). Alternatively, polynucleotides may be modified to include uracil and the nucleobases removed with Uracil-DNA Glycosylase (UDG). In one embodiment, the one or more spacers do not comprise any abasic nucleotides.

### Anchors

In some embodiments, the double stranded nucleic acid, construct thereof, or an adapter attached thereto comprises a membrane anchor or a transmembrane pore anchor e.g. attached to the adapter. In one embodiment the anchor aids in characterisation of the double stranded nucleic acid in accordance with the methods disclosed herein. For example, a membrane anchor or transmembrane pore anchor may promote localisation of the double stranded nucleic acid around a nanopore in a membrane.

The anchor may be a polypeptide anchor and/or a hydrophobic anchor that can be inserted into the membrane. In one embodiment, the hydrophobic anchor is a lipid, fatty acid, sterol, carbon nanotube, polypeptide, protein or amino acid, for example cholesterol, palmitate or tocopherol. The anchor may comprise thiol, biotin or a surfactant.

In one aspect the anchor may be biotin (for binding to streptavidin), amylose (for binding to maltose binding protein or a fusion protein), Ni-NTA (for binding to poly-histidine or poly-histidine tagged proteins) or peptides (such as an antigen).

In one embodiment, the anchor comprises a linker, or 2, 3, 4 or more linkers. Preferred linkers include, but are not limited to, polymers, such as polynucleotides, polyethylene glycols (PEGs), polysaccharides and polypeptides. These linkers may be linear, branched or circular. For instance, the linker may be a circular polynucleotide. The adapter may hybridise to a complementary sequence on a circular polynucleotide linker. The one or more anchors or one or more linkers may comprise a component that can be cut or broken down, such as a restriction site or a photolabile group. The linker may be functionalised with maleimide groups to attach to cysteine residues in proteins. Suitable linkers are described in WO 2010/086602.

In one embodiment, the anchor is cholesterol or a fatty acyl chain. For example, any fatty acyl chain having a length of from 6 to 30 carbon atom, such as hexadecanoic acid, may be used. Examples of suitable anchors and methods of attaching anchors to adapters are disclosed in WO 2012/164270 and WO 2015/150786.

### Controlling Movement of the Analyte with respect to a detector

As explained in more detail above, some embodiments of the methods provided herein comprise contacting the cross-linked construct with an enzyme which sequentially processes the first strand and the second strand of the construct. In some embodiments the enzyme is used to control the movement of the construct with respect to a detector such as a transmembrane nanopore. In some embodiments the enzyme is used to process the construct in a next generation sequencing method, such as a sequencing by synthesis method, e.g. single-molecule real-time sequencing.

In some embodiments, the enzyme is capable of sequentially processing the first strand and the second strand of the cross-linked construct. In some embodiments the enzyme is capable of processing the first strand and then processing the second strand. In some embodiments the enzyme is capable of processing the first strand, the inter-strand cross-link and the second strand. In some embodiments the enzyme is capable of processing from the first strand through the inter-strand cross-link to the second strand. In some embodiments the enzyme is capable of sequentially processing the first strand and the second strand without processing through the inter-strand cross-link.

The movement of the construct with respect to the detector used in the sequencing technique may be controlled by any suitable means. In some embodiments, the movement of the construct is driven by a physical or chemical force (potential). In some embodiments the physical force is provided by an electrical (e.g. voltage) potential or a temperature gradient, etc.

In some embodiments, the detector is a nanopore and the construct moves with respect to the nanopore as an electrical potential is applied across the nanopore. Polynucleotides are negatively charged, and so applying a voltage potential across a nanopore will cause the polynucleotides to move with respect to the nanopore under the influence of the applied voltage potential. For example, if a positive voltage potential is applied to the *trans* side of the nanopore relative to the *cis* side of the nanopore, then this will induce a negatively charged analyte to move from the *cis* side of the nanopore to the *trans* side of the nanopore. Similarly, if a positive voltage potential is applied to the *trans* side of the nanopore relative to the *cis* side of the nanopore then this will impede the movement of a negatively charged analyte from the *trans* side of the nanopore to the *cis* side of the nanopore. The opposite will occur if a negative voltage potential is applied to the *trans* side of the nanopore relative to the *cis* side of the nanopore. Apparatuses and methods of applying appropriate voltages are described in more detail herein.

In some embodiments the chemical force is provided by a concentration (e.g. pH) gradient.

In some embodiments, the methods provided herein comprise contacting the construct with a polynucleotide-processing enzyme.

Suitable polynucleotide-processing enzymes are also sometimes known as motor proteins or polynucleotide-handling enzymes. Suitable polynucleotide-processing enzymes are known in the art. In some embodiments, therefore, the provided methods comprise contacting the construct with a motor protein, wherein the motor protein controls the movement of the construct with respect to a nanopore.

In some embodiments, a motor protein can be present on the construct prior to its contact with a nanopore. For example, a motor protein can be present on an adapter comprising part of the construct analyte, or can be otherwise present on a portion of the construct.

In some embodiments, the motor protein is modified to prevent the motor protein disengaging from the polynucleotide, construct or adapter (other than by passing off the end of the polynucleotide/construct/adapter). Such modified motor proteins are particularly suitable for use in the disclosed methods.

The motor protein can be adapted in any suitable way. For example, the motor protein can be loaded onto the polynucleotide, construct or adapter and then modified in order to prevent it from disengaging. Alternatively, the motor protein can be modified to prevent it from disengaging before it is loaded onto the polynucleotide, construct or adapter. Modification of a motor protein in order to prevent it from disengaging from a polynucleotide, construct or adapter can be achieved using methods known in the art, such as those discussed in WO 2014/013260, and with particular reference to passages describing the modification of motor proteins (polynucleotide binding proteins) such as helicases in order to prevent them from disengaging with polynucleotide strands.

For example, the motor protein may have a polynucleotide-unbinding opening; e.g. a cavity, cleft or void through which a polynucleotide strand may pass when the motor protein disengages from the strand. In some embodiments, the polynucleotide-unbinding opening for a given motor protein (polynucleotide binding protein) can be determined by reference to its structure, e.g. by reference to its X-ray crystal structure. The X-ray crystal structure may be obtained in the presence and/or the absence of a polynucleotide substrate. In some embodiments, the location of a polynucleotide-unbinding opening in a given motor protein may be deduced or confirmed by molecular modelling using standard packages known in the art. In some embodiments, the polynucleotide-unbinding opening may be transiently produced by movement of one or more parts e.g. one or more domains of the motor protein.

The motor protein may be modified by closing the polynucleotide-unbinding opening. Closing the polynucleotide-unbinding opening may therefore prevent the motor protein from disengaging from the polynucleotide or adapter. For example, the motor protein may be modified by covalently closing the polynucleotide-unbinding opening. In some embodiments, a motor protein for addressing in this way is a helicase, as described herein.

In one embodiment, a motor protein is or is derived from a polynucleotide handling enzyme. A polynucleotide handling enzyme is a polypeptide that is capable of interacting with and modifying at least one property of a polynucleotide. The enzyme may modify the polynucleotide by cleaving it to form individual nucleotides or shorter chains of nucleotides, such as di- or trinucleotides. The enzyme may modify the polynucleotide by orienting it or moving it to a specific position.

The motor protein may be chosen or selected according to the double stranded nucleic acid target to be sequenced in the methods disclosed herein. Alternatively, the double stranded nucleic acid target may be chosen or selected according to the motor protein used (if any) to process the first strand and the second strand of the terminally cross-linked construct. For example, typically DNA motor protein can be used when the target double stranded nucleic acid is double stranded DNA. RNA motor protein can be used when the target double stranded nucleic acid is double stranded RNA. Motor proteins which can process both DNA and RNA can be used when the target double stranded nucleic acid is a DNA-RNA hybrid.

In one embodiment, the motor protein is derived from a member of any of the Enzyme Classification (EC) groups 3.1.11, 3.1.13, 3.1.14, 3.1.15, 3.1.16, 3.1.21, 3.1.22, 3.1.25, 3.1.26, 3.1.27, 3.1.30 and 3.1.31.

In some embodiments of the claimed methods, the motor protein is a helicase, a polymerase, an exonuclease, a topoisomerase, or a variant thereof.

In one embodiment, the motor protein is an exonuclease. Suitable enzymes include, but are not limited to, exonuclease I from *E. coli* (SEQ ID NO: 1), exonuclease III enzyme from *E. coli* (SEQ ID NO: 2), RecJ from *T. thermophilus* (SEQ ID NO: 3) and bacteriophage lambda exonuclease (SEQ ID NO: 4), TatD exonuclease and variants thereof. Three subunits comprising the sequence shown in SEQ ID NO: 3 or a variant thereof interact to form a trimer exonuclease.

In one embodiment, the motor protein is a polymerase. The polymerase may be PyroPhage^{®} 3173 DNA Polymerase (which is commercially available from Lucigen^{®} Corporation), SD Polymerase (commercially available from Bioron^{®}), Klenow from NEB or variants thereof. In one embodiment, the enzyme is Phi29 DNA polymerase (SEQ ID NO: 5) or a variant thereof. Modified versions of Phi29 polymerase that may be used in the disclosed methods are disclosed in US Patent No. 5,576,204.

In embodiments of the methods provided herein which comprise sequencing the construct using a sequencing by synthesis reaction, the enzyme is typically a polymerase, e.g. a polymerase as described herein.

In one embodiment the motor protein is a topoisomerase. In one embodiment, the topoisomerase is a member of any of the Moiety Classification (EC) groups 5.99.1.2 and 5.99.1.3. The topoisomerase may be a reverse transcriptase, which are enzymes capable of catalysing the formation of cDNA from a RNA template. They are commercially available from, for instance, New England Biolabs^{®} and Invitrogen^{®}.

In one embodiment, the motor protein is a helicase. Any suitable helicase can be used in accordance with the methods provided herein. For example, the or each motor protein used in accordance with the present disclosure may be independently selected from a Hel308 helicase, a RecD helicase, a TraI helicase, a TrwC helicase, an XPD helicase, and a Dda helicase, or a variant thereof. Monomeric helicases may comprise several domains attached together. For instance, TraI helicases and TraI subgroup helicases may contain two RecD helicase domains, a relaxase domain and a C-terminal domain. The domains typically form a monomeric helicase that is capable of functioning without forming oligomers. Particular examples of suitable helicases include Hel308, NS3, Dda, UvrD, Rep, PcrA, Pif1 and TraI. These helicases typically work on single stranded DNA. Examples of helicases that can move along both strands of a double stranded DNA include FtfK and hexameric enzyme complexes, or multisubunit complexes such as RecBCD. NS3 helicases are particularly suitable for use in the disclosed methods as they are capable of processing both DNA and RNA and so can be used in embodiments of the disclosed methods in which the target double stranded nucleic acid is a DNA-RNA hybrid.

Hel308 helicases are described in publications such as WO 2013/057495, RecD helicases are described in publications such as WO 2013/098562, XPD helicases are described in publications such as WO 2013/098561. Dda helicases are described in publications such as WO 2015/055981 and WO 2016/055777

In one embodiment the helicase comprises the sequence shown in SEQ ID NO: 6 (Trwc Cba) or a variant thereof, the sequence shown in SEQ ID NO: 7 (Hel308 Mbu) or a variant thereof or the sequence shown in SEQ ID NO: 8 (Dda) or a variant thereof. Variants may differ from the native sequences in any of the ways discussed herein. An example variant of SEQ ID NO: 8 comprises E94C/A360C. A further example variant of SEQ ID NO: 8 comprises E94C/A360C and then (ΔM1)G1G2 (i.e. deletion of M1 and then addition of G1 and G2).

In some embodiments a motor protein (e.g. a helicase) can control the movement of a construct in at least two active modes of operation (when the motor protein is provided with all the necessary components to facilitate movement, e.g. fuel and cofactors such as ATP and Mg²⁺ discussed herein) and one inactive mode of operation (when the motor protein is not provided with the necessary components to facilitate movement).

When provided with all the necessary components to facilitate movement (i.e. in the active modes), the motor protein (e.g. helicase) moves along the polynucleotide construct in a 5' to 3' or a 3' to 5' direction (depending on the motor protein). In embodiments in which the motor protein is used to control the movement of a polynucleotide strand of the construct with respect to a nanopore, the motor protein can be used to either move the construct away from (e.g. out of) the pore (e.g. against an applied force) or the construct towards (e.g. into) the pore (e.g. with an applied force). For example, when the end of the construct towards which the motor protein moves is captured by a pore, the motor protein works against the direction of the force and pulls the threaded construct out of the pore (e.g. into the *cis* chamber). However, when the end away from which the motor protein moves is captured in the pore, the motor protein works with the direction of the force and pushes the threaded construct into the pore (e.g. into the *trans* chamber).

When the motor protein (e.g. helicase) is not provided with the necessary components to facilitate movement (i.e. in the inactive mode) it can bind to the construct and act as a brake slowing the movement of the construct when it is moved with respect to a nanopore, e.g. by being pulled into the pore by a force. In the inactive mode, it does not matter which end of the construct is captured, it is the applied force which determines the movement of the construct with respect to the pore, and the polynucleotide binding protein acts as a brake. When in the inactive mode, the movement control of the construct by the polynucleotide binding protein can be described in a number of ways including ratcheting, sliding and braking.

A motor protein typically requires fuel in order to handle the processing of polynucleotides. Fuel is typically free nucleotides or free nucleotide analogues. The free nucleotides may be one or more of, but are not limited to, adenosine monophosphate (AMP), adenosine diphosphate (ADP), adenosine triphosphate (ATP), guanosine monophosphate (GMP), guanosine diphosphate (GDP), guanosine triphosphate (GTP), thymidine monophosphate (TMP), thymidine diphosphate (TDP), thymidine triphosphate (TTP), uridine monophosphate (UMP), uridine diphosphate (UDP), uridine triphosphate (UTP), cytidine monophosphate (CMP), cytidine diphosphate (CDP), cytidine triphosphate (CTP), cyclic adenosine monophosphate (cAMP), cyclic guanosine monophosphate (cGMP), deoxyadenosine monophosphate (dAMP), deoxyadenosine diphosphate (dADP), deoxyadenosine triphosphate (dATP), deoxyguanosine monophosphate (dGMP), deoxyguanosine diphosphate (dGDP), deoxyguanosine triphosphate (dGTP), deoxythymidine monophosphate (dTMP), deoxythymidine diphosphate (dTDP), deoxythymidine triphosphate (dTTP), deoxyuridine monophosphate (dUMP), deoxyuridine diphosphate (dUDP), deoxyuridine triphosphate (dUTP), deoxycytidine monophosphate (dCMP), deoxycytidine diphosphate (dCDP) and deoxycytidine triphosphate (dCTP). The free nucleotides are usually selected from AMP, TMP, GMP, CMP, UMP, dAMP, dTMP, dGMP or dCMP. The free nucleotides are typically adenosine triphosphate (ATP).

A cofactor for the motor protein is a factor that allows the motor protein to function. The cofactor is preferably a divalent metal cation. The divalent metal cation is preferably Mg²⁺, Mn²⁺, Ca²⁺ or Co²⁺. The cofactor is most preferably Mg²⁺.

### Nanopore

As explained above, some embodiments of the methods provided herein comprise sequencing the cross-linked construct using a nanopore sensor.

In embodiments of the disclosed methods which relate to a nanopore, any suitable nanopore can be used. In one embodiment a nanopore is a transmembrane pore.

A transmembrane pore is a structure that crosses the membrane to some degree. It permits hydrated ions driven by an applied potential to flow across or within the membrane. The transmembrane pore typically crosses the entire membrane so that hydrated ions may flow from one side of the membrane to the other side of the membrane. However, the transmembrane pore does not have to cross the membrane. It may be closed at one end. For instance, the pore may be a well, gap, channel, trench or slit in the membrane along which or into which hydrated ions may flow.

Any transmembrane pore may be used in the methods provided herein. The pore may be biological or artificial. Suitable pores include, but are not limited to, protein pores, polynucleotide pores and solid state pores. The pore may be a DNA origami pore (Langecker et al., Science, 2012; 338: 932-936). Suitable DNA origami pores are disclosed in WO2013/083983.

In one embodiment, the nanopore is a transmembrane protein pore. A transmembrane protein pore is a polypeptide or a collection of polypeptides that permits hydrated ions, such as polynucleotide, to flow from one side of a membrane to the other side of the membrane. In the methods provided herein, the transmembrane protein pore is capable of forming a pore that permits hydrated ions driven by an applied potential to flow from one side of the membrane to the other. The transmembrane protein pore preferably permits polynucleotides to flow from one side of the membrane, such as a triblock copolymer membrane, to the other. The transmembrane protein pore allows a polynucleotide to be moved through the pore.

In one embodiment, the nanopore is a transmembrane protein pore which is a monomer or an oligomer. The pore is preferably made up of several repeating subunits, such as at least 6, at least 7, at least 8, at least 9, at least 10, at least 11, at least 12, at least 13, at least 14, at least 15, or at least 16 subunits. The pore is preferably a hexameric, heptameric, octameric or nonameric pore. The pore may be a homo-oligomer or a heterooligomer.

In one embodiment, the transmembrane protein pore comprises a barrel or channel through which the ions may flow. The subunits of the pore typically surround a central axis and contribute strands to a transmembrane β-barrel or channel or a transmembrane α-helix bundle or channel.

Typically, the barrel or channel of the transmembrane protein pore comprises amino acids that facilitate interaction with an analyte, such as a target polynucleotide (as described herein). These amino acids are preferably located near a constriction of the barrel or channel. The transmembrane protein pore typically comprises one or more positively charged amino acids, such as arginine, lysine or histidine, or aromatic amino acids, such as tyrosine or tryptophan. These amino acids typically facilitate the interaction between the pore and nucleotides, polynucleotides or nucleic acids.

In one embodiment, the nanopore is a transmembrane protein pore derived from β-barrel pores or α-helix bundle pores. β-barrel pores comprise a barrel or channel that is formed from β-strands. Suitable β-barrel pores include, but are not limited to, β-toxins, such as α-hemolysin, anthrax toxin and leukocidins, and outer membrane proteins/porins of bacteria, such as *Mycobacterium smegmatis* porin (Msp), for example MspA, MspB, MspC or MspD, CsgG, outer membrane porin F (OmpF), outer membrane porin G (OmpG), outer membrane phospholipase A and *Neisseria* autotransporter lipoprotein (NalP) and other pores, such as lysenin. α-helix bundle pores comprise a barrel or channel that is formed from α-helices. Suitable α-helix bundle pores include, but are not limited to, inner membrane proteins and α outer membrane proteins, such as WZA and ClyA toxin.

In one embodiment the nanopore is a transmembrane pore derived from or based on Msp, α-hemolysin (α-HL), lysenin, CsgG, ClyA, Sp1 or haemolytic protein fragaceatoxin C (FraC).

In one embodiment, the nanopore is a transmembrane protein pore derived from CsgG, e.g. from CsgG from *E. coli* Str. K-12 substr. MC4100. Such a pore is oligomeric and typically comprises 7, 8, 9 or 10 monomers derived from CsgG. The pore may be a homo-oligomeric pore derived from CsgG comprising identical monomers. Alternatively, the pore may be a hetero-oligomeric pore derived from CsgG comprising at least one monomer that differs from the others. Examples of suitable pores derived from CsgG are disclosed in WO 2016/034591.

In one embodiment, the nanopore is a transmembrane pore derived from lysenin. Examples of suitable pores derived from lysenin are disclosed in WO 2013/153359.

In one embodiment, the nanopore is a transmembrane pore derived from or based on α-hemolysin (α-HL). The wild type α-hemolysin pore is formed of 7 identical monomers or sub-units (i.e., it is heptameric). An α-hemolysin pore may be α-hemolysin-NN or a variant thereof. The variant preferably comprises N residues at positions E111 and K147.

In one embodiment, the nanopore is a transmembrane protein pore derived from Msp, e.g. from MspA. Examples of suitable pores derived from MspA are disclosed in WO 2012/107778.

In one embodiment, the nanopore is a transmembrane pore derived from or based on ClyA.

### Tags

In some embodiments of the methods provided herein, as explained above, sequencing the cross-linked double stranded nucleic acid construct comprises contacting the construct with a transmembrane nanopore. In some embodiments, a tag on the nanopore can be used, e.g. to promote the capture of the analyte by the nanopore.

The interaction between a tag on a nanopore and the binding site on a polynucleotide (*e.g*., the binding site present in an adaptor attached to a polynucleotide, wherein the binding site can be provided by an anchor or a leader sequence of an adaptor or by a capture sequence within the duplex stem of an adaptor) may be reversible. For example, a polynucleotide can bind to a tag on a nanopore, *e.g*., via its adaptor, and release at some point, *e.g.,* during characterization of the polynucleotide by the nanopore and/or during processing by a motor protein. A strong non-covalent bond (*e.g*., biotin/avidin) is still reversible and can be useful in some embodiments of the methods described herein. For example, a pair of pore tag and polynucleotide adaptor can be designed to provide a sufficient interaction between the complement of a double stranded polynucleotide (or a portion of an adaptor that is attached to the complement) and the nanopore such that the complement is held close to the nanopore (without detaching from the nanopore and diffusing away) but is able to release from the nanopore as it is processed.

A pore tag and polynucleotide adaptor can be configured such that the binding strength or affinity of a binding site on the polynucleotide (*e.g.*, a binding site provided by an anchor or a leader sequence of an adaptor or by a capture sequence within the duplex stem of an adaptor) to a tag on a nanopore is sufficient to maintain the coupling between the nanopore and polynucleotide until an applied force is placed on it to release the bound polynucleotide from the nanopore. In some embodiments where the analyte is a double stranded polynucleotide, the applied force may be processing of a complement strand by a polymerase.

In some embodiments, the tags or tethers are uncharged. This can ensure that the tags or tethers are not drawn into the nanopore under the influence of a potential difference if present.

One or more molecules that attract or bind the polynucleotide or adaptor may be linked to the detector (e.g. the pore). Any molecule that hybridizes to the adaptor and/or target polynucleotide may be used. The molecule attached to the pore may be selected from a PNA tag, a PEG linker, a short oligonucleotide, a positively charged amino acid and an aptamer. Pores having such molecules linked to them are known in the art. For example, pores having short oligonucleotides attached thereto are disclosed in Howarka et al (2001) Nature Biotech. 19: 636-639 and WO 2010/086620, and pores comprising PEG attached within the lumen of the pore are disclosed in Howarka et al (2000) J. Am. Chem. Soc. 122(11): 2411-2416.

A short oligonucleotide attached to the nanopore, which comprises a sequence complementary to a sequence in the leader sequence or another single stranded sequence in an adaptor may be used to enhance capture of the target polynucleotide in the methods described herein.

In some embodiments, the tag or tether may comprise or be an oligonucleotide *(e.g.,* DNA, RNA, LNA, BNA, PNA, or morpholino). The oligonucleotide can have about 10-30 nucleotides in length or about 10-20 nucleotides in length. In some embodiments, the oligonucleotide can have at least one end *(e.g.,* 3'- or 5'-end) modified for conjugation to other modifications or to a solid substrate surface including, *e.g.,* a bead. The end modifiers may add a reactive functional group which can be used for conjugation. Examples of functional groups that can be added include, but are not limited to amino, carboxyl, thiol, maleimide, aminooxy, and any combinations thereof. The functional groups can be combined with different length of spacers *(e.g.,* C3, C9, C12, Spacer 9 and 18) to add physical distance of the functional group from the end of the oligonucleotide sequence.

Examples of modifications on the 3' and/or 5' end of oligonucleotides include, but are not limited to 3' affinity tag and functional groups for chemical linkage (including, *e.g*., 3'-biotin, 3'-primary amine, 3'-disulfide amide, 3'-pyridyl dithio, and any combinations thereof); 5' end modifications (including, *e.g*., 5'-primary ammine, and/or 5'-dabcyl), modifications for click chemistry (including, *e.g*., 3'-azide, 3'-alkyne, 5'-azide, 5'-alkyne), and any combinations thereof.

In some embodiments, the tag or tether may further comprise a polymeric linker, *e.g.,* to facilitate coupling to the nanopore. An exemplary polymeric linker includes, but is not limited to polyethylene glycol (PEG). The polymeric linker may have a molecular weight of about 500 Da to about 10 kDa (inclusive), or about 1 kDa to about 5 kDa (inclusive). The polymeric linker *(e.g.,* PEG) can be functionalized with different functional groups including, *e.g*., but not limited to maleimide, NHS ester, dibenzocyclooctyne (DBCO), azide, biotin, amine, alkyne, aldehyde, and any combinations thereof.

Other examples of a tag or tether include, but are not limited to His tags, biotin or streptavidin, antibodies that bind to analytes, aptamers that bind to analytes, analyte binding domains such as DNA binding domains (including, *e.g*., peptide zippers such as leucine zippers, single-stranded DNA binding proteins (SSB)), and any combinations thereof.

The tag or tether may be attached to the external surface of a nanopore, *e.g*., on the *cis* side of a membrane, using any methods known in the art. For example, one or more tags or tethers can be attached to the nanopore via one or more cysteines (cysteine linkage), one or more primary amines such as lysines, one or more non-natural amino acids, one or more histidines (His tags), one or more biotin or streptavidin, one or more antibody-based tags, one or more enzyme modification of an epitope (including, *e.g*., acetyl transferase), and any combinations thereof. Suitable methods for carrying out such modifications are well-known in the art. Suitable non-natural amino acids include, but are not limited to, 4-azido-L-phenylalanine (Faz) and any one of the amino acids numbered 1-71 in Figure 1 of Liu C. C. and Schultz P. G., Annu. Rev. Biochem., 2010, 79, 413-444.

In some embodiments where one or more tags or tethers are attached to a nanopore via cysteine linkage(s), the one or more cysteines can be introduced to one or more monomers that form the nanopore by substitution. In some embodiments, the nanopore may be chemically modified by attachment of (i) Maleimides including diabromomaleimides such as: 4-phenylazomaleinanil, 1.N-(2-Hydroxyethyl)maleimide, N-Cyclohexylmaleimide, 1.3-Maleimidopropionic Acid, 1.1-4-Aminophenyl-1H-pyrrole,2,5,dione, 1.1-4-Hydroxyphenyl-1H-pyrrole,2,5,dione, N-Ethylmaleimide, N-Methoxycarbonylmaleimide, N-tert-Butylmaleimide, N-(2-Aminoethyl)maleimide , 3-Maleimido-PROXYL , N-(4-Chlorophenyl)maleimide, 1-[4-(dimethylamino)-3,5-dinitrophenyl]-1H-pyrrole-2,5-dione, N-[4-(2-Benzimidazolyl)phenyl]maleimide, N-[4-(2-benzoxazolyl)phenyl]maleimide, N-(1-naphthyl)-maleimide, N-(2,4-xylyl)maleimide, N-(2,4-difluorophenyl)maleimide , N-(3-chloro-para-tolyl)-maleimide, 1-(2-amino-ethyl)-pyrrole-2,5-dione hydrochloride, 1-cyclopentyl-3-methyl-2,5-dihydro-1H-pyrrole-2,5-dione, 1-(3-aminopropyl)-2,5-dihydro-1H-pyrrole-2,5-dione hydrochloride, 3-methyl-1-[2-oxo-2-(piperazin-1-yl)ethyl]-2,5-dihydro-1H-pyrrole-2,5-dione hydrochloride, 1-benzyl-2,5-dihydro-1H-pyrrole-2,5-dione, 3-methyl-1-(3,3,3-trifluropropyl)-2,5-dihydro-1H-pyrrole-2,5-dione, 1-[4-(methylamino)cyclohexyl]-2,5-dihydro-1H-pyrrole-2,5-dione trifluroacetic acid, SMILES O=C1C=CC(=O)N1CC=2C=CN=CC2, SMILES O=C1C=CC(=O)N1CN2CCNCC2, 1-benzyl-3-methyl-2,5-dihydro-1H-pyrrole-2,5-dione, 1-(2-fluorophenyl)-3-methyl-2,5-dihydro 1H-pyrrole-2,5-dione, N-(4-phenoxyphenyl)maleimide , N-(4-nitrophenyl)maleimide (ii) Iodocetamides such as :3-(2-Iodoacetamido)-proxyl, N-(cyclopropylmethyl)-2-iodoacetamide, 2-iodo-N-(2-phenylethyl)acetamide, 2-iodo-N-(2,2,2-trifluoroethyl)acetamide, N-(4-acetylphenyl)-2-iodoacetamide, N-(4-(aminosulfonyl)phenyl)-2-iodoacetamide, N-(1,3-benzothiazol-2-yl)-2-iodoacetamide, N-(2,6-diethylphenyl)-2-iodoacetamide, N-(2-benzoyl-4-chlorophenyl)-2-iodoacetamide, (iii) Bromoacetamides: such as N-(4-(acetylamino)phenyl)-2-bromoacetamide , N-(2-acetylphenyl)-2-bromoacetamide , 2-bromo-n-(2-cyanophenyl)acetamide, 2-bromo-N-(3-(trifluoromethyl)phenyl)acetamide, N-(2-benzoylphenyl)-2-bromoacetamide , 2-bromo-N-(4-fluorophenyl)-3-methylbutanamide, N-Benzyl-2-bromo-N-phenylpropionamide, N-(2-bromo-butyryl)-4-chloro-benzenesulfonamide, 2-Bromo-N-methyl-N-phenylacetamide, 2-bromo-N-phenethyl-acetamide,2-adamantan-1-yl-2-bromo-N-cyclohexyl-acetamide, 2-bromo-N-(2-methylphenyl)butanamide, Monobromoacetanilide, (iv) Disulphides such as: aldrithiol-2 , aldrithiol-4 , isopropyl disulfide, 1-(Isobutyldisulfanyl)-2-methylpropane, Dibenzyl disulfide, 4-aminophenyl disulfide, 3-(2-Pyridyldithio)propionic acid, 3-(2-Pyridyldithio)propionic acid hydrazide, 3-(2-Pyridyldithio)propionic acid N-succinimidyl ester, am6amPDP1-βCD and (v) Thiols such as: 4-Phenylthiazole-2-thiol, Purpald, 5,6,7,8-tetrahydro-quinazoline-2-thiol.

In some embodiments, the tag or tether may be attached directly to a nanopore or via one or more linkers. The tag or tether may be attached to the nanopore using the hybridization linkers described in WO 2010/086602. Alternatively, peptide linkers may be used. Peptide linkers are amino acid sequences. The length, flexibility and hydrophilicity of the peptide linker are typically designed such that it does not to disturb the functions of the monomer and pore. Preferred flexible peptide linkers are stretches of 2 to 20, such as 4, 6, 8, 10 or 16, serine and/or glycine amino acids. More preferred flexible linkers include (SG)₁, (SG)₂, (SG)₃, (SG)₄, (SG)₅ and (SG)₈ wherein S is serine and G is glycine. Preferred rigid linkers are stretches of 2 to 30, such as 4, 6, 8, 16 or 24, proline amino acids. More preferred rigid linkers include (P)₁₂ wherein P is proline.

### Membrane

In embodiments of the disclosed methods which comprise the use of a transmembrane nanopore, the transmembrane nanopore is typically present in a membrane. Any suitable membrane may be used in the system.

The membrane is preferably an amphiphilic layer. An amphiphilic layer is a layer formed from amphiphilic molecules, such as phospholipids, which have both hydrophilic and lipophilic properties. The amphiphilic molecules may be synthetic or naturally occurring. Non-naturally occurring amphiphiles and amphiphiles which form a monolayer are known in the art and include, for example, block copolymers (Gonzalez-Perez et al., Langmuir, 2009, 25, 10447-10450). Block copolymers are polymeric materials in which two or more monomer sub-units that are polymerized together to create a single polymer chain. Block copolymers typically have properties that are contributed by each monomer sub-unit. However, a block copolymer may have unique properties that polymers formed from the individual sub-units do not possess. Block copolymers can be engineered such that one of the monomer sub-units is hydrophobic (i.e. lipophilic), whilst the other sub-unit(s) are hydrophilic whilst in aqueous media. In this case, the block copolymer may possess amphiphilic properties and may form a structure that mimics a biological membrane. The block copolymer may be a diblock (consisting of two monomer subunits), but may also be constructed from more than two monomer sub-units to form more complex arrangements that behave as amphipiles. The copolymer may be a triblock, tetrablock or pentablock copolymer. The membrane is preferably a triblock copolymer membrane.

Archaebacterial bipolar tetraether lipids are naturally occurring lipids that are constructed such that the lipid forms a monolayer membrane. These lipids are generally found in extremophiles that survive in harsh biological environments, thermophiles, halophiles and acidophiles. Their stability is believed to derive from the fused nature of the final bilayer. It is straightforward to construct block copolymer materials that mimic these biological entities by creating a triblock polymer that has the general motif hydrophilic-hydrophobic-hydrophilic. This material may form monomeric membranes that behave similarly to lipid bilayers and encompass a range of phase behaviours from vesicles through to laminar membranes. Membranes formed from these triblock copolymers hold several advantages over biological lipid membranes. Because the triblock copolymer is synthesised, the exact construction can be carefully controlled to provide the correct chain lengths and properties required to form membranes and to interact with pores and other proteins.

Block copolymers may also be constructed from sub-units that are not classed as lipid sub-materials; for example a hydrophobic polymer may be made from siloxane or other non-hydrocarbon based monomers. The hydrophilic sub-section of block copolymer can also possess low protein binding properties, which allows the creation of a membrane that is highly resistant when exposed to raw biological samples. This head group unit may also be derived from non-classical lipid head-groups.

Triblock copolymer membranes also have increased mechanical and environmental stability compared with biological lipid membranes, for example a much higher operational temperature or pH range. The synthetic nature of the block copolymers provides a platform to customise polymer based membranes for a wide range of applications.

In some embodiments, the membrane is one of the membranes disclosed in International Application No. WO2014/064443 or WO2014/064444.

The amphiphilic molecules may be chemically-modified or functionalised to facilitate coupling of the polynucleotide. The amphiphilic layer may be a monolayer or a bilayer. The amphiphilic layer is typically planar. The amphiphilic layer may be curved. The amphiphilic layer may be supported.

Amphiphilic membranes are typically naturally mobile, essentially acting as two dimensional fluids with lipid diffusion rates of approximately 10⁻⁸ cm s⁻¹. This means that the pore and coupled polynucleotide can typically move within an amphiphilic membrane.

The membrane may be a lipid bilayer. Lipid bilayers are models of cell membranes and serve as excellent platforms for a range of experimental studies. For example, lipid bilayers can be used for *in vitro* investigation of membrane proteins by single-channel recording. Alternatively, lipid bilayers can be used as biosensors to detect the presence of a range of substances. The lipid bilayer may be any lipid bilayer. Suitable lipid bilayers include, but are not limited to, a planar lipid bilayer, a supported bilayer or a liposome. The lipid bilayer is preferably a planar lipid bilayer. Suitable lipid bilayers are disclosed in WO 2008/102121, WO 2009/077734 and WO 2006/100484.

Methods for forming lipid bilayers are known in the art. Lipid bilayers are commonly formed by the method of Montal and Mueller (Proc. Natl. Acad. Sci. USA., 1972; 69: 3561-3566), in which a lipid monolayer is carried on aqueous solution/air interface past either side of an aperture which is perpendicular to that interface. The lipid is normally added to the surface of an aqueous electrolyte solution by first dissolving it in an organic solvent and then allowing a drop of the solvent to evaporate on the surface of the aqueous solution on either side of the aperture. Once the organic solvent has evaporated, the solution/air interfaces on either side of the aperture are physically moved up and down past the aperture until a bilayer is formed. Planar lipid bilayers may be formed across an aperture in a membrane or across an opening into a recess.

The method of Montal & Mueller is popular because it is a cost-effective and relatively straightforward method of forming good quality lipid bilayers that are suitable for protein pore insertion. Other common methods of bilayer formation include tip-dipping, painting bilayers and patch-clamping of liposome bilayers.

Tip-dipping bilayer formation entails touching the aperture surface (for example, a pipette tip) onto the surface of a test solution that is carrying a monolayer of lipid. Again, the lipid monolayer is first generated at the solution/air interface by allowing a drop of lipid dissolved in organic solvent to evaporate at the solution surface. The bilayer is then formed by the Langmuir-Schaefer process and requires mechanical automation to move the aperture relative to the solution surface.

For painted bilayers, a drop of lipid dissolved in organic solvent is applied directly to the aperture, which is submerged in an aqueous test solution. The lipid solution is spread thinly over the aperture using a paintbrush or an equivalent. Thinning of the solvent results in formation of a lipid bilayer. However, complete removal of the solvent from the bilayer is difficult and consequently the bilayer formed by this method is less stable and more prone to noise during electrochemical measurement.

Patch-clamping is commonly used in the study of biological cell membranes. The cell membrane is clamped to the end of a pipette by suction and a patch of the membrane becomes attached over the aperture. The method has been adapted for producing lipid bilayers by clamping liposomes which then burst to leave a lipid bilayer sealing over the aperture of the pipette. The method requires stable, giant and unilamellar liposomes and the fabrication of small apertures in materials having a glass surface.

Liposomes can be formed by sonication, extrusion or the Mozafari method (Colas et al. (2007) Micron 38:841-847).

In some embodiments, a lipid bilayer is formed as described in International Application No. WO 2009/077734. Advantageously in this method, the lipid bilayer is formed from dried lipids. In a most preferred embodiment, the lipid bilayer is formed across an opening as described in WO2009/077734.

A lipid bilayer is formed from two opposing layers of lipids. The two layers of lipids are arranged such that their hydrophobic tail groups face towards each other to form a hydrophobic interior. The hydrophilic head groups of the lipids face outwards towards the aqueous environment on each side of the bilayer. The bilayer may be present in a number of lipid phases including, but not limited to, the liquid disordered phase (fluid lamellar), liquid ordered phase, solid ordered phase (lamellar gel phase, interdigitated gel phase) and planar bilayer crystals (lamellar sub-gel phase, lamellar crystalline phase).

Any lipid composition that forms a lipid bilayer may be used. The lipid composition is chosen such that a lipid bilayer having the required properties, such surface charge, ability to support membrane proteins, packing density or mechanical properties, is formed. The lipid composition can comprise one or more different lipids. For instance, the lipid composition can contain up to 100 lipids. The lipid composition preferably contains 1 to 10 lipids. The lipid composition may comprise naturally-occurring lipids and/or artificial lipids.

The lipids typically comprise a head group, an interfacial moiety and two hydrophobic tail groups which may be the same or different. Suitable head groups include, but are not limited to, neutral head groups, such as diacylglycerides (DG) and ceramides (CM); zwitterionic head groups, such as phosphatidylcholine (PC), phosphatidylethanolamine (PE) and sphingomyelin (SM); negatively charged head groups, such as phosphatidylglycerol (PG); phosphatidylserine (PS), phosphatidylinositol (PI), phosphatic acid (PA) and cardiolipin (CA); and positively charged headgroups, such as trimethylammonium-Propane (TAP). Suitable interfacial moieties include, but are not limited to, naturally-occurring interfacial moieties, such as glycerol-based or ceramidebased moieties. Suitable hydrophobic tail groups include, but are not limited to, saturated hydrocarbon chains, such as lauric acid (*n*-Dodecanolic acid), myristic acid (*n-*Tetradecononic acid), palmitic acid (*n*-Hexadecanoic acid), stearic acid (*n*-Octadecanoic) and arachidic (*n*-Eicosanoic); unsaturated hydrocarbon chains, such as oleic acid (*cis*-9-Octadecanoic); and branched hydrocarbon chains, such as phytanoyl. The length of the chain and the position and number of the double bonds in the unsaturated hydrocarbon chains can vary. The length of the chains and the position and number of the branches, such as methyl groups, in the branched hydrocarbon chains can vary. The hydrophobic tail groups can be linked to the interfacial moiety as an ether or an ester. The lipids may be mycolic acid.

The lipids can also be chemically-modified. The head group or the tail group of the lipids may be chemically-modified. Suitable lipids whose head groups have been chemically-modified include, but are not limited to, PEG-modified lipids, such as 1,2-Diacyl-sn-Glycero-3-Phosphoethanolamine-N -[Methoxy(Polyethylene glycol)-2000]; functionalised PEG Lipids, such as 1,2-Distearoyl-sn-Glycero-3 Phosphoethanolamine-N-[Biotinyl(Polyethylene Glycol)2000]; and lipids modified for conjugation, such as 1,2-Dioleoyl-sn-Glycero-3-Phosphoethanolamine-N-(succinyl) and 1,2-Dipalmitoyl-sn-Glycero-3-Phosphoethanolamine-N-(Biotinyl). Suitable lipids whose tail groups have been chemically-modified include, but are not limited to, polymerisable lipids, such as 1,2-bis(10,12-tricosadiynoyl)-sn-Glycero-3-Phosphocholine; fluorinated lipids, such as 1-Palmitoyl-2-(16-Fluoropalmitoyl)-sn-Glycero-3-Phosphocholine; deuterated lipids, such as 1,2-Dipalmitoyl-D62-sn-Glycero-3-Phosphocholine; and ether linked lipids, such as 1,2-Di-O-phytanyl-sn-Glycero-3-Phosphocholine. The lipids may be chemically-modified or functionalised to facilitate coupling of the polynucleotide.

The amphiphilic layer, for example the lipid composition, typically comprises one or more additives that will affect the properties of the layer. Suitable additives include, but are not limited to, fatty acids, such as palmitic acid, myristic acid and oleic acid; fatty alcohols, such as palmitic alcohol, myristic alcohol and oleic alcohol; sterols, such as cholesterol, ergosterol, lanosterol, sitosterol and stigmasterol; lysophospholipids, such as 1-Acyl-2-Hydroxy-sn- Glycero-3-Phosphocholine; and ceramides.

In another embodiment, the membrane comprises a solid state layer. Solid state layers can be formed from both organic and inorganic materials including, but not limited to, microelectronic materials, insulating materials such as Si₃N₄, Al₂O₃, and SiO, organic and inorganic polymers such as polyamide, plastics such as Teflon^{®} or elastomers such as two-component addition-cure silicone rubber, and glasses. The solid state layer may be formed from graphene. Suitable graphene layers are disclosed in WO 2009/035647. If the membrane comprises a solid state layer, the pore is typically present in an amphiphilic membrane or layer contained within the solid state layer, for instance within a hole, well, gap, channel, trench or slit within the solid state layer. The skilled person can prepare suitable solid state/amphiphilic hybrid systems. Suitable systems are disclosed in WO 2009/020682 and WO 2012/005857. Any of the amphiphilic membranes or layers discussed above may be used.

The methods disclosed herein are typically carried out using (i) an artificial amphiphilic layer comprising a pore, (ii) an isolated, naturally-occurring lipid bilayer comprising a pore, or (iii) a cell having a pore inserted therein. The methods are typically carried out using an artificial amphiphilic layer, such as an artificial triblock copolymer layer. The layer may comprise other transmembrane and/or intramembrane proteins as well as other molecules in addition to the pore. Suitable apparatus and conditions are discussed below. The disclosed methods are typically carried out *in vitro.*

### Conditions

As explained above, some exemplary embodiments of the present disclosure relate to sequencing a target double stranded nucleic acid as it moves with respect to, such as into or through, a transmembrane nanopore as described in more detail herein.

The characterisation methods may be carried out using any apparatus that is suitable for investigating a membrane/pore system in which a pore is inserted into a membrane. The characterisation method may be carried out using any apparatus that is suitable for transmembrane pore sensing. For example, the apparatus may comprise a chamber comprising an aqueous solution and a barrier that separates the chamber into two sections. The barrier may have an aperture in which a membrane containing a transmembrane pore is formed. Transmembrane pores are described herein.

The characterisation methods may be carried out using the apparatus described in WO 2008/102120, WO 2010/122293 or WO 00/28312.

The characterisation methods may involve measuring the ion current flow through the pore, typically by measurement of a current. Alternatively, the ion flow through the pore may be measured optically, such as disclosed by Heron et al: J. Am. Chem. Soc. 9 Vol. 131, No. 5, 2009. Therefore the apparatus may also comprise an electrical circuit capable of applying a potential and measuring an electrical signal across the membrane and pore. The characterisation methods may be carried out using a patch clamp or a voltage clamp. The characterisation methods preferably involve the use of a voltage clamp. The characterisation methods may be carried out on a silicon-based array of wells where each array comprises 128, 256, 512, 1024, 2000, 3000, 4000, 6000, 10000, 12000, 15000 or more wells.

The characterisation methods may involve the measuring of a current flowing through the pore. The method is typically carried out with a voltage applied across the membrane and pore. The voltage used is typically from +2 V to -2 V, typically -400 mV to +400mV. The voltage used is preferably in a range having a lower limit selected from -400 mV, -300 mV, -200 mV, -150 mV, -100 mV, -50 mV, -20mV and 0 mV and an upper limit independently selected from -10 mV, + 20 mV, +50 mV, +100 mV, +150 mV, +200 mV, +300 mV and +400 mV. The voltage used is more preferably in the range 100 mV to 240mV and most preferably in the range of 120 mV to 220 mV. It is possible to increase discrimination between different nucleotides by a pore by using an increased applied potential.

The characterisation methods are typically carried out in the presence of any charge carriers, such as metal salts, for example alkali metal salts, halide salts, for example chloride salts, such as alkali metal chloride salt. Charge carriers may include ionic liquids or organic salts, for example tetramethyl ammonium chloride, trimethylphenyl ammonium chloride, phenyltrimethyl ammonium chloride, or 1-ethyl-3-methyl imidazolium chloride. In the exemplary apparatus discussed above, the salt is present in the aqueous solution in the chamber. Potassium chloride (KCl), sodium chloride (NaCl) or caesium chloride (CsCl) is typically used. KCl is preferred. The salt may be an alkaline earth metal salt such as calcium chloride (CaCl₂). The salt concentration may be at saturation. The salt concentration may be 3M or lower and is typically from 0.1 to 2.5 M, from 0.3 to 1.9 M, from 0.5 to 1.8 M, from 0.7 to 1.7 M, from 0.9 to 1.6 M or from 1 M to 1.4 M. The salt concentration is preferably from 150 mM to 1 M. The characterisation method is preferably carried out using a salt concentration of at least 0.3 M, such as at least 0.4 M, at least 0.5 M, at least 0.6 M, at least 0.8 M, at least 1.0 M, at least 1.5 M, at least 2.0 M, at least 2.5 M or at least 3.0 M. High salt concentrations provide a high signal to noise ratio and allow for currents indicative of binding/no binding to be identified against the background of normal current fluctuations.

The characterisation methods are typically carried out in the presence of a buffer. In the exemplary apparatus discussed above, the buffer is present in the aqueous solution in the chamber. Any suitable buffer may be used. Typically, the buffer is HEPES. Another suitable buffer is Tris-HCl buffer. The methods are typically carried out at a pH of from 4.0 to 12.0, from 4.5 to 10.0, from 5.0 to 9.0, from 5.5 to 8.8, from 6.0 to 8.7 or from 7.0 to 8.8 or 7.5 to 8.5. The pH used is preferably about 7.5.

The characterisation methods may be carried out at from 0 °C to 100 °C, from 15 °C to 95 °C, from 16 °C to 90 °C, from 17 °C to 85 °C, from 18 °C to 80 °C, 19 °C to 70 °C, or from 20 °C to 60 °C. The characterisation methods are typically carried out at room temperature. The characterisation methods are optionally carried out at a temperature that supports enzyme function, such as about 37 °C.

### Other Methods

Also disclosed herein is a method of sequencing a target double stranded nucleic acid comprising:
(a') forming an inter-strand cross-link between the first and second strands of the target double stranded nucleic acid by exposing the target double stranded nucleic acid to a cross-linking agent;
(b') allowing the target double stranded nucleic acid to break at or in the vicinity of the inter-strand cross-link to form at least one double stranded nucleic acid construct cross-linked at or in the vicinity of the terminus of the construct; and
(c') sequencing the cross-linked construct formed in (b) using a single molecule sequencing technique.

Preferred embodiments of such methods comprise contacting the cross-linked construct with an enzyme which reads through sequentially processes the first strand and the second strand of the terminally cross-linked construct formed in (a'). In some embodiments this provides orthogonal proof-reading sequence information. In some embodiments the enzyme processes the cross-link. In some embodiments the enzyme does not process the cross-link. In some embodiments the enzyme is a polynucleotide-processing enzyme disclosed herein.

Such methods are particularly suited to sequencing the cross-linked construct using sequencing-by-synthesis techniques such as single-molecule real-time sequencing based on the detection of base incorporation catalysed by enzymes such as DNA polymerases. Suitable polymerases are described herein.

In such methods, the double stranded nucleic acid, inter-strand cross-link, and sequencing techniques are typically as described herein. Breakage of double stranded nucleic acids is described in more detail herein.

### Construct

Also provided herein are cross-linked nucleic acid constructs formed by exposing a double stranded nucleic acid to a cross-linking agent as described herein. The double stranded nucleic acid and cross-linking agent are preferably as described in more detail herein.

The construct provided herein may further comprise one or more adapters and/or anchors and/or spacers as described herein. The construct may comprise a polynucleotide-processing enzyme as described herein attached thereto.

### System

Also provided are systems comprising a cross-linked nucleic acid constructs formed by exposing a double stranded nucleic acid to a cross-linking agent as described herein; one or more polynucleotide-processing enzymes as described herein and one or more nanopores. Typically such systems are provided for characterising the double stranded nucleic acid.

In one embodiment provided is a system for characterising a target double stranded nucleic acid comprising:
- a construct formed by exposing the target double stranded nucleic acid to a cross-linking agent;
- a polynucleotide-processing enzyme; and
- a nanopore for characterising the target polynucleotide as the target polynucleotide moves with respect to the nanopore.

Typically, the construct, enzyme and nanopore are as described in more detail herein.

### Kit

Also provided are kits comprising one or more cross-linking reagents and one or more polynucleotide-processing enzymes. In some embodiments the kit provided herein further comprises one or more adapters and/or anchors and/or spacers as described herein. In some embodiments the cross-linking reagent(s) and polynucleotide-processing enzymes are typically as described herein.

In some embodiments the kit further comprises a detector for sequencing the construct formed by contacting a target double stranded nucleic acid with the cross-linking reagent(s). In some embodiments the detector is a nanopore as described herein.

In some embodiments the kit further comprises reagents such as buffer solutions, fuel molecules (e.g. those described above, such as nucleoside triphosphates, e.g. ATP), and instructions for use.

The kit may be configured for use with an algorithm, also provided herein, adapted to be run on a computer system. The algorithm may be adapted to detect sequence information from a first strand of a double stranded nucleic acid attached to a second strand of the double stranded nucleic acid by an inter-strand cross-link, and to selectively process the signal obtained as the first strand and the second strand are sequentially sequenced, e.g. using a nanopore sensor, so as to obtain orthogonal proof-reading sequence information. Also provided is a system comprising computing means configured to detect sequence information from a first strand of a double stranded nucleic acid attached to a second strand of the double stranded nucleic acid by an inter-strand cross-link and to selectively process the signal obtained as the first strand and the second strand are sequentially sequenced, e.g. using a nanopore sensor, so as to obtain orthogonal proof-reading sequence information. In some embodiments the system comprises receiving means for receiving data from detection of the first and second strands of the cross-linked double stranded nucleic acid, processing means for processing the signal obtained as the first strand and the second strand are sequentially sequenced, and output means for outputting the proof-read sequence information.
The following examples are provided to better illustrate particular embodiments, and they should not be considered limiting the application. The application is limited only by the claims.

### EXAMPLES

This example shows that double stranded nucleic acids can be cross-linked in accordance with the methods disclosed herein and that the inter-strand cross-linked construct thereby formed can be sequenced using single-molecule sequencing.

Two samples of Lambda genomic DNA were prepared for sequencing. Sample A was a control sample, comprising 1000ng of Lambda DNA (obtained from New England Biolabs, product NEB N3013), which was not subjected to inter-strand cross-linking prior to sequencing. Sample B, also comprising 1000ng of Lambda DNA (NEB N3013), was incubated on ice under a UV lamp at 365 nm for 8 minutes.

Both samples were separately prepared for DNA sequencing. The DNA samples were first end repaired and dA-tailed using NEBNext^{®} Ultra^{™} II End Repair/dA-Tailing Module (New England Biolabs, product NEB E7546) following the manufacturers guidelines. The samples were then cleaned up with a 1 x SPRI purification using Agencourt AMPure beads (Beckman Coulter) following the manufacturers guidelines.

Strands were subjected to pipette shearing during sample preparation, particularly during the dAtailing and SPRI steps.

The samples were then adapted for sequencing using the Oxford Nanopore Technologies sequencing kit (Oxford Nanopore Technologies, product SQK-LSK108), following the manufacturers guidelines. This attaches Sequencing Adapters preloaded with an E8 motor to the dA-tailed ends of the dsDNA in the samples via ligation. The prepared samples were then run on Oxford Nanopore MinION flowcells using baseline sequencing protocols in accordance with the manufacturer's guidelines.

The electrical current vs. time data was analysed from bulk raw data sequencing files. Figure 5 shows typical example sections of the current (*y* axis, in pA) versus time (*x* axis, in s) data, highlighting the events corresponding to sequencing strands. Figure 5 shows examples of sequenced template-complement constructs, with sections marked "A" for template and "B" for the linked reverse complement.

Strands with both template and reverse complement sections are sometimes evident by changes in the current signal between the two sections, which may occur when the template and reverse-complement sections re-anneal on the opposite side of the nanopore during translocation. A change in current level can provide a useful indication of the sequencing of the two linked strands. However, a change in current level is not required in the methods disclosed herein. Depending on the sequence of the double stranded nucleic acid sequenced in the methods disclosed herein, a change in current level may or may not be observed on sequencing the inter-strand cross-linked construct. Thus, whilst a change in current level may be a useful indicator, a lack of a change in current level is not an indicator that the method has not worked.

Data corresponding to separate sequencing strand events were extracted and basecalled using Oxford Nanopore's Guppy basecallers, and aligned to the full Lambda phage genome reference using conventional sequence alignment tools. Strands with aligned template and reverse-complement were identified as "2D", and strands with template only sections as "1D". The table below shows the percentage of strands identified as 2D for both the cross-linked sample and the control. It is clear that by crosslinking the double stranded nucleic acid sample in accordance with the methods provided herein, the percentage of strands identified as 2D was significantly increased.

| **Sample** | **Number of strands analysed** | **% identified as 2D by alignment to reference genome** |
|---|---|---|
| Sample A (Lambda DNA control) | 7729 | 7.3% |
| Sample B (Crosslinked Lambda DNA) | 2868 | 57.8% |

Figure 6 shows the signal and the sequence alignment of both the template and linked reverse-complement sections of some example 2D strands. As explained above, the difference in template and reverse-complement signal is typically evident in the current vs time signal. However, even where no such change in current signal is observed the basecall alignments confirm that strands contain both template and reverse-complement sections.

### Description of the Sequence Listing

SEQ ID NO: 1 shows the amino acid sequence of (hexa-histidine tagged) exonuclease I (EcoExo I) from *E. coli.*
SEQ ID NO: 2 shows the amino acid sequence of the exonuclease III enzyme from *E. coli.*
SEQ ID NO: 3 shows the amino acid sequence of the RecJ enzyme from *T. thermophilus* (TthRecJ-cd).
SEQ ID NO: 4 shows the amino acid sequence of bacteriophage lambda exonuclease. The sequence is one of three identical subunits that assemble into a trimer. (http://www.neb.com/nebecomm/products/productM0262.asp).
SEQ ID NO: 5 shows the amino acid sequence of Phi29 DNA polymerase from *Bacillus subtilis.*
SEQ ID NO: 6 shows the amino acid sequence of Trwc Cba (*Citromicrobium bathyomarinum*) helicase.
SEQ ID NO: 7 shows the amino acid sequence of Hel308 Mbu (*Methanococcoides burtonii*) helicase .
SEQ ID NO: 8 shows the amino acid sequence of the Dda helicase 1993 from Enterobacteria phage T4.

### SEQUENCE LISTING

**SEQ ID NO: 1 - exonuclease I from *E. coli*** 
MMNDGKQQSTFLFHDYETFGTHPALDRPAQFAAIRTDSEFNVIGEPEVFYCKPADDYLPQ
PGAVLITGITPQEARAKGENEAAFAARIHSLFTVPKTCILGYNNVRFDDEVTRNIFYRNF
YDPYAWSWQHDNSRWDLLDVMRACYALRPEGINWPENDDGLPSFRLEHLTKANGIEHSNA
HDAMADVYATIAMAKLVKTRQPRLFDYLFTHRNKHKLMALIDVPQMKPLVHVSGMFGAWR
GNTSWVAPLAWHPENRNAVIMVDLAGDISPLLELDSDTLRERLYTAKTDLGDNAAVPVKL
VHINKCPVLAQANTLRPEDADRLGINRQHCLDNLKILRENPQVREKVVAIFAEAEPFTPS
DNVDAQLYNGFFSDADRAAMKIVLETEPRNLPALDITFVDKRIEKLLFNYRARNFPGTLD
YAEQQRWLEHRRQVFTPEFLQGYADELQMLVQQYADDKEKVALLKALWQYAEEIVSGSGH
HHHHH
**SEQ ID NO: 2 - exonuclease III enzyme from *E. coli*** 
MKFVSFNINGLRARPHQLEAIVEKHQPDVIGLQETKVHDDMFPLEEVAKLGYNVFYHGQK
GHYGVALLTKETPIAVRRGFPGDDEEAQRRIIMAEIPSLLGNVTVINGYFPQGESRDHPI
KFPAKAQFYQNLQNYLETELKRDNPVLIMGDMNISPTDLDIGIGEENRKRWLRTGKCSFL
PEEREWMDRLMSWGLVDTFRHANPQTADRFSWFDYRSKGFDDNRGLRIDLLLASQPLAEC
CVETGIDYEIRSMEKPSDHAPVWATFRR
**SEQ ID NO: 3 - RecJ enzyme from T. thermophilus** 
MFRRKEDLDPPLALLPLKGLREAAALLEEALRQGKRIRVHGDYDADGLTGTAILVRGLAA
LGADVHPFIPHRLEEGYGVLMERVPEHLEASDLFLTVDCGITNHAELRELLENGVEVIVT
DHHTPGKTPPPGLVVHPALTPDLKEKPTGAGVAFLLLWALHERLGLPPPLEYADLAAVGT
IADVAPLWGWNRALVKEGLARIPASSWVGLRLLAEAVGYTGKAVEVAFRIAPRINAASRL
GEAEKALRLLLTDDAAEAQALVGELHRLNARRQTLEEAMLRKLLPQADPEAKAIVLLDPE
GHPGVMGIVASRILEATLRPVFLVAQGKGTVRSLAPISAVEALRSAEDLLLRYGGHKEAA
GFAMDEALFPAFKARVEAYAARFPDPVREVALLDLLPEPGLLPQVFRELALLEPYGEGNP
EPLFL
**SEQ ID NO: 4 - bacteriophage lambda exonuclease** 
MTPDIILQRTGIDVRAVEQGDDAWHKLRLGVITASEVHNVIAKPRSGKKWPDMKMSYFHT
LLAEVCTGVAPEVNAKALAWGKQYENDARTLFEFTSGVNVTESPIIYRDESMRTACSPDG
LCSDGNGLELKCPFTSRDFMKFRLGGFEAIKSAYMAQVQYSMWVTRKNAWYFANYDPRMK
REGLHYVVIERDEKYMASFDEIVPEFIEKMDEALAEIGFVFGEQWR
**SEQ ID NO: 5 - Phi29 DNA polymerase** 
MKHMPRKMYSCAFETTTKVEDCRVWAYGYMNIEDHSEYKIGNSLDEFMAWVLKVQADLYF
HNLKFDGAFIINWLERNGFKWSADGLPNTYNTIISRMGQWYMIDICLGYKGKRKIHTVIY
DSLKKLPFPVKKIAKDFKLTVLKGDIDYHKERPVGYKITPEEYAYIKNDIQIIAEALLIQ
FKQGLDRMTAGSDSLKGFKDIITTKKFKKVFPTLSLGLDKEVRYAYRGGFTWLNDRFKEK
EIGEGMVFDVNSLYPAQMYSRLLPYGEPIVFEGKYVWDEDYPLHIQHIRCEFELKEGYIP
TIQIKRSRFYKGNEYLKSSGGEIADLWLSNVDLELMKEHYDLYNVEYISGLKFKATTGLF
KDFIDKWTYIKTTSEGAIKQLAKLMLNSLYGKFASNPDVTGKVPYLKENGALGFRLGEEE
TKDPVYTPMGVFITAWARYTTITAAQACYDRIIYCDTDSIHLTGTEIPDVIKDIVDPKKL
GYWAHESTFKRAKYLRQKTYIQDIYMKEVDGKLVEGSPDDYTDIKFSVKCAGMTDKIKKE
VTFENFKVGFSRKMKPKPVQVPGGVVLVDDTFTIKSGGSAWSHPQFEKGGGSGGGSGGSA
WSHPQFEK
**SEQ ID NO: 6 - Trwc Cba helicase** 
MLSVANVRSPSAAASYFASDNYYASADADRSGQWIGDGAKRLGLEGKVEARAFDALLRGE
LPDGSSVGNPGQAHRPGTDLTFSVPKSWSLLALVGKDERIIAAYREAVVEALHWAEKNAA
ETRVVEKGMVVTQATGNLAIGLFQHDTNRNQEPNLHFHAVIANVTQGKDGKWRTLKNDRL
WQLNTTLNSIAMARFRVAVEKLGYEPGPVLKHGNFEARGISREQVMAFSTRRKEVLEARR
GPGLDAGRIAALDTRASKEGIEDRATLSKQWSEAAQSIGLDLKPLVDRARTKALGQGMEA
TRIGSLVERGRAWLSRFAAHVRGDPADPLVPPSVLKQDRQTIAAAQAVASAVRHLSQREA
AFERTALYKAALDFGLPTTIADVEKRTRALVRSGDLIAGKGEHKGWLASRDAVVTEQRIL
SEVAAGKGDSSPAITPQKAAASVQAAALTGQGFRLNEGQLAAARLILISKDRTIAVQGIA
GAGKSSVLKPVAEVLRDEGHPVIGLAIQNTLVQMLERDTGIGSQTLARFLGGWNKLLDDP
GNVALRAEAQASLKDHVLVLDEASMVSNEDKEKLVRLANLAGVHRLVLIGDRKQLGAVDA
GKPFALLQRAGIARAEMATNLRARDPVVREAQAAAQAGDVRKALRHLKSHTVEARGDGAQ
VAAETWLALDKETRARTSIYASGRAIRSAVNAAVQQGLLASREIGPAKMKLEVLDRVNTT
 
REELRHLPAYRAGRVLEVSRKQQALGLFIGEYRVIGQDRKGKLVEVEDKRGKRFRFDPAR
IRAGKGDDNLTLLEPRKLEIHEGDRIRWTRNDHRRGLFNADQARVVEIANGKVTFETSKG
DLVELKKDDPMLKRIDLAYALNVHMAQGLTSDRGIAVMDSRERNLSNQKTFLVTVTRLRD
HLTLVVDSADKLGAAVARNKGEKASAIEVTGSVKPTATKGSGVDQPKSVEANKAEKELTR
SKSKTLDFGI
**SEQ ID NO: 7 - He1308 Mbu helicase** 
MMIRELDIPRDIIGFYEDSGIKELYPPQAEAIEMGLLEKKNLLAAIPTASGKTLLAELAM
IKAIREGGKALYIVPLRALASEKFERFKELAPFGIKVGISTGDLDSRADWLGVNDIIVAT
SEKTDSLLRNGTSWMDEITTVVVDEIHLLDSKNRGPTLEVTITKLMRLNPDVQVVALSAT
VGNAREMADWLGAALVLSEWRPTDLHEGVLFGDAINFPGSQKKIDRLEKDDAVNLVLDTI
KAEGQCLVFESSRRNCAGFAKTASSKVAKILDNDIMIKLAGIAEEVESTGETDTAIVLAN
CIRKGVAFHHAGLNSNHRKLVENGFRQNLIKVISSTPTLAAGLNLPARRVIIRSYRRFDS
NFGMQPIPVLEYKQMAGRAGRPHLDPYGESVLLAKTYDEFAQLMENYVEADAEDIWSKLG
TENALRTHVLSTIVNGFASTRQELFDFFGATFFAYQQDKWMLEEVINDCLEFLIDKAMVS
ETEDIEDASKLFLRGTRLGSLVSMLYIDPLSGSKIVDGFKDIGKSTGGNMGSLEDDKGDD
ITVTDMTLLHLVCSTPDMRQLYLRNTDYTIVNEYIVAHSDEFHEIPDKLKETDYEWFMGE
VKTAMLLEEWVTEVSAEDITRHFNVGEGDIHALADTSEWLMHAAAKLAELLGVEYSSHAY
SLEKRIRYGSGLDLMELVGIRGVGRVRARKLYNAGFVSVAKLKGADISVLSKLVGPKVAY
NILSGIGVRVNDKHFNSAPISSNTLDTLLDKNQKTFNDFQ
**SEQ ID NO: 8 - Dda helicase**

MTFDDLTEGQKNAFNIVMKAIKEKKHHVTINGPAGTGKTTLTKFIIEALISTGETGIILA
APTHAAKKILSKLSGKEASTIHSILKINPVTYEENVLFEQKEVPDLAKCRVLICDEVSMY
DRKLFKILLSTIPPWCTIIGIGDNKQIRPVDPGENTAYISPFFTHKDFYQCELTEVKRSN
APIIDVATDVRNGKWIYDKVVDGHGVRGFTGDTALRDFMVNYFSIVKSLDDLFENRVMAF
TNKSVDKLNSIIRKKIFETDKDFIVGEIIVMQEPLFKTYKIDGKPVSEIIFNNGQLVRII
EAEYTSTFVKARGVPGEYLIRHWDLTVETYGDDEYYREKIKIISSDEELYKFNLFLGKTA
ETYKNWNKGGKAPWSDFWDAKSQFSKVKALPASTFHKAQGMSVDRAFIYTPCIHYADVEL
AQQLLYVGVTRGRYDVFYV

## Claims

1. A method of sequencing a target double stranded nucleic acid comprising:
(a) forming an inter-strand cross-link between the first and second strands of the target double stranded nucleic acid by exposing the target double stranded nucleic acid to a crosslinking agent; thereby forming at least one cross-linked double stranded nucleic acid construct; and
(b) sequencing the cross-linked construct formed in (a) using a single molecule sequencing technique.

2. A method according to claim 1, further comprising allowing the double stranded nucleic acid to break at or in the vicinity of the inter-strand cross-link formed in step (a) so that the inter-strand cross-link is positioned at or in the vicinity of the terminus of the construct.

3. A method according to claim 2, wherein the break at or in the vicinity of the inter-strand cross-link occurs naturally due to distortion or is induced by physical agitation.

4. A method according to any one of the preceding claims, wherein the single molecule sequencing technique comprises contacting the cross-linked construct with an enzyme which sequentially processes the first strand and the second strand of the crosslinked construct formed; preferably wherein said sequencing technique provides orthogonal proof-reading sequence information.

5. A method according to any one of the preceding claims, wherein the cross-linking agent is or comprises electromagnetic radiation.

6. A method according to any one of the preceding claims, wherein the cross-linking agent is or comprises a chemical reagent.

7. A method according to any one of the preceding claims, wherein the cross-linking agent is or comprises a nucleic acid cross-linking enzyme.

8. A method according to any of the preceding claims, wherein the inter-strand cross-link is formed at a random position on the target double stranded nucleic acid.

9. A method according to any of claims 1 to 7, wherein the inter-strand cross-link is targeted to a particular base composition on the target double stranded nucleic acid.

10. A method according to any of the preceding claims, wherein the inter-strand cross-link is formed between nucleobases in the first and second strands of the target double stranded nucleic acid.

11. A method according to any of claims 1 to 9, wherein the inter-strand cross-link is formed between sugar groups in the first and second strands of the target double stranded nucleic acid.

12. A method according to any of claims 1 to 9, wherein the inter-strand cross-link is formed between nucleobases in the first and second strands of the target double stranded nucleic acid and between sugar groups in the first and second strands of the target double stranded nucleic acid.

13. A method according to any one of claims 1 to 9 wherein the inter-strand cross-link is formed between nucleobase adducts in the first and second strands of the target double stranded nucleic acid.

14. A method according to any of the preceding claims wherein the cross-link is positioned within 100 bases of the terminus of the cross-linked construct formed in part (b) of claim 1.

15. A method according to any of the preceding claims wherein the single molecule sequencing technique comprises sequencing using a nanopore sensor.

## Patentansprüche

1. Verfahren zum Sequenzieren einer doppelsträngigen Zielnukleinsäure, umfassend:
(a) Bilden einer Zwischenstrangvernetzung zwischen dem ersten und dem zweiten Strang der doppelsträngigen Zielnukleinsäure, indem die doppelsträngige Zielnukleinsäure einem Vernetzungsmittel ausgesetzt wird, wodurch mindestens ein vernetztes doppelsträngiges Nukleinsäurekonstrukt gebildet wird; und
(b) Sequenzieren des in (a) gebildeten vernetzten Konstrukts unter Verwendung einer Einzelmolekülsequenzierungstechnik.

2. Verfahren nach Anspruch 1, ferner umfassend das Erlauben, dass die doppelsträngige Nukleinsäure an oder in der Nähe der in Schritt (a) gebildeten Zwischenstrangvernetzung bricht, sodass die Zwischenstrangvernetzung an oder in der Nähe des Terminus des Konstrukts positioniert wird.

3. Verfahren nach Anspruch 2, wobei der Bruch an oder in der Nähe der Zwischenstrangvernetzung auf natürliche Weise durch Verformung auftritt oder durch physikalische Bewegung hervorgerufen wird.

4. Verfahren nach einem der vorstehenden Ansprüche, wobei die Einzelmolekülsequenzierungstechnik das Inkontaktbringen des vernetzten Konstrukts mit einem Enzym umfasst, das den ersten Strang und den zweiten Strang des gebildeten vernetzten Konstrukts sequentiell verarbeitet; wobei die Sequenzierungstechnik vorzugsweise orthogonale Korrekturlesesequenzinformationen bereitstellt.

5. Verfahren nach einem der vorstehenden Ansprüche, wobei das Vernetzungsmittel elektromagnetische Strahlung ist oder umfasst.

6. Verfahren nach einem der vorstehenden Ansprüche, wobei das Vernetzungsmittel ein chemisches Reagenz ist oder umfasst.

7. Verfahren nach einem der vorstehenden Ansprüche, wobei das Vernetzungsmittel ein Nukleinsäurevernetzungsenzym ist oder umfasst.

8. Verfahren nach einem der vorstehenden Ansprüche, wobei die Zwischenstrangvernetzung an einer zufälligen Position auf der doppelsträngigen Zielnukleinsäure gebildet wird.

9. Verfahren nach einem der Ansprüche 1 bis 7, wobei die Zwischenstrangvernetzung auf eine bestimmte Basiszusammensetzung auf der Zieldoppelstrangnukleinsäure ausgerichtet ist.

10. Verfahren nach einem der vorstehenden Ansprüche, wobei die Zwischenstrangvernetzung zwischen Nukleobasen im ersten und zweiten Strang der doppelsträngigen Zielnukleinsäure gebildet wird.

11. Verfahren nach einem der Ansprüche 1 bis 9, wobei die Zwischenstrangvernetzung zwischen Zuckergruppen im ersten und zweiten Strang der doppelsträngigen Zielnukleinsäure gebildet wird.

12. Verfahren nach einem der Ansprüche 1 bis 9, wobei die Zwischenstrangvernetzung zwischen Nukleobasen im ersten und zweiten Strang der Zieldoppelstrangnukleinsäure und zwischen Zuckergruppen im ersten und zweiten Strang der Zieldoppelstrangnukleinsäure gebildet wird.

13. Verfahren nach einem der Ansprüche 1 bis 9, wobei die Zwischenstrangvernetzung zwischen Nukleobasenaddukten im ersten und zweiten Strang der doppelsträngigen Zielnukleinsäure gebildet wird.

14. Verfahren nach einem der vorstehenden Ansprüche, wobei die Vernetzung innerhalb von 100 Basen des Terminus des in Teil (b) von Anspruch 1 gebildeten vernetzten Konstrukts positioniert wird.

15. Verfahren nach einem der vorstehenden Ansprüche, wobei die Einzelmolekülsequenzierungstechnik die Sequenzierung unter Verwendung eines Nanoporensensors umfasst.

## Revendications

1. Procédé de séquençage d'un acide nucléique cible à double brin comprenant :
(a) la formation d'une liaison de réticulation interbrins entre le premier et le second brins de l'acide nucléique cible à double brin par exposition de l'acide nucléique cible à double brin à un agent de réticulation, formant ainsi au moins une construction d'acide nucléique à double brin réticulé ; et
(b) le séquençage de la construction réticulée formée à l'étape (a) à l'aide d'une technique de séquençage de molécule unique.

2. Procédé selon la revendication 1, comprenant en outre l'autorisation de la rupture de l'acide nucléique à double brin au niveau ou à proximité de la liaison de réticulation interbrins formée à l'étape (a), de sorte que la liaison de réticulation interbrins soit située au niveau ou à proximité de l'extrémité de la construction.

3. Procédé selon la revendication 2, dans lequel la rupture au niveau ou à proximité de la liaison de réticulation interbrins se produit naturellement du fait d'une distorsion ou est induite par une agitation physique.

4. Procédé selon l'une quelconque des revendications précédentes, dans lequel la technique de séquençage de molécule unique comprend la mise en contact de la construction réticulée avec une enzyme qui traite séquentiellement le premier brin et le second brin de la construction réticulée formée ; de préférence, dans lequel ladite technique de séquençage fournit des informations de séquence de relecture orthogonale.

5. Procédé selon l'une quelconque des revendications précédentes, dans lequel l'agent de réticulation est ou comprend un rayonnement électromagnétique.

6. Procédé selon l'une quelconque des revendications précédentes, dans lequel l'agent de réticulation est ou comprend un réactif chimique.

7. Procédé selon l'une quelconque des revendications précédentes, dans lequel l'agent de réticulation est ou comprend une enzyme de réticulation d'acide nucléique.

8. Procédé selon l'une quelconque des revendications précédentes, dans lequel la liaison de réticulation interbrins est formée à une position aléatoire sur l'acide nucléique cible à double brin.

9. Procédé selon l'une quelconque des revendications 1 à 7, dans lequel la liaison de réticulation interbrins est ciblée vers une composition de base particulière sur l'acide nucléique cible à double brin.

10. Procédé selon l'une quelconque des revendications précédentes, dans lequel la liaison de réticulation interbrins est formée entre des nucléobases du premier brin et du second brin de l'acide nucléique cible à double brin.

11. Procédé selon l'une quelconque des revendications 1 à 9, dans lequel la liaison de réticulation interbrins est formée entre des groupements sucre du premier et du second brins de l'acide nucléique cible à double brin.

12. Procédé selon l'une quelconque des revendications 1 à 9, dans lequel la liaison de réticulation interbrins est formée entre des nucléobases du premier et du second brins de l'acide nucléique cible à double brin et entre des groupements sucre du premier et du second brins de l'acide nucléique cible à double brin.

13. Procédé selon l'une quelconque des revendications 1 à 9, dans lequel la liaison de réticulation interbrins est formée entre des adduits de nucléobases du premier et du second brins de l'acide nucléique cible à double brin.

14. Procédé selon l'une quelconque des revendications précédentes, dans lequel la liaison de réticulation est située à moins de 100 bases de l'extrémité de la construction réticulée formée à l'étape (b) de la revendication 1.

15. Procédé selon l'une quelconque des revendications précédentes, dans lequel la technique de séquençage de molécule unique comprend un séquençage à l'aide d'un capteur à nanopore.
